(19) Europäisches Patentamt
European Patent Office
Office européen des brevets

(11) Veröffentlichungsnummer : **0 057 357**
**B1**

# (12) EUROPÄISCHE PATENTSCHRIFT

(45) Veröffentlichungstag der Patentschrift : **25.07.84**

(21) Anmeldenummer : **82100251.6**

(22) Anmeldetag : **15.01.82**

(51) Int. Cl.[3] : **A 01 N 57/20**, A 01 N 53/00, C 07 D249/08, C 07 D233/60 // (A01N57/20, 43/64, 43/50),(A01N53/00, 43/64, 43/50)

## (54) Mittel zur Hemmung des Pflanzenwachstums.

(30) Priorität : **27.01.81 DE 3102588**

(43) Veröffentlichungstag der Anmeldung : **11.08.82 Patentblatt 82/32**

(45) Bekanntmachung des Hinweises auf die Patenterteilung : **25.07.84 Patentblatt 84/30**

(84) Benannte Vertragsstaaten : **AT BE CH DE FR GB IT LI NL SE**

(56) Entgegenhaltungen :
**EP-A- 0 005 600**
**RESEARCH DISCLOSURE, Nr. 176, Dezember 1978, Seiten 44-47, Nr. 17652, Homewell, Havant, Hampshire, G.B."Compounds of general formula", Seite 47, Absatz 9, Zeile 17**

(73) Patentinhaber : **BAYER AG**
**Konzernverwaltung RP Patentabteilung**
**D-5090 Leverkusen 1 Bayerwerk (DE)**

(72) Erfinder : **Lürssen, Klaus, Dr.**
**August-Kierspel-Strasse 89**
**D-5060 Bergisch-Gladbach 2 (DE)**
Erfinder : **Holmwood, Graham, Dr.**
**KaternbergerStrasse 184**
**D-5600 Wuppertal 1 (DE)**
Erfinder : **Krämer, Wolfgang, Dr.**
**Am Eckbusch 30/45**
**D-5600 Wuppertal 1 (DE)**
Erfinder : **Regel, Erik, Ing.-grad.**
**Bergerheide 72a**
**D-5600 Wuppertal 1 (DE)**
Erfinder : **Reiser, Wolf, Dr.**
**Klebitzweg 12a**
**D-5600 Wuppertal 1 (DE)**
Erfinder : **Schröder, Rolf, Dr.**
**Pahlkestrasse 17**
**D-5600 Wuppertal 1 (DE)**

**EP 0 057 357 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Wirkstoffkombinationen, die aus bekannten Azolen bzw. bekannten Pyrimidinbutanol-Derivaten einerseits und bekannten Phosphonsäure-Derivaten bzw. 1-Aminocyclopropan-1-carbonsäure-Derivaten andererseits bestehen, und sehr gut zur Hemmung des Pflanzenwachstums geeignet sind.

Es ist bereits bekannt geworden, daß zahlreiche Triazol-Derivate pflanzenwachstumsreguliegende Eigenschaften besitzen (vgl. DE-A 2 407 143, DE-A 2 737 489, DE-A 2 904 061, DE-A 2 645 617, DE-A 2 838 847 und JP-A 53 130 661). Die Wirksamkeit dieser Stoffe ist jedoch, vor allem bei niedrigen Aufwandmengen, nicht immer befriedigend.

Weiterhin ist bereits bekannt, daß bestimmte Pyrimidin-butanol-Derivate zur Regulierung des Pflanzenwachstums verwendbar sind (vgl. DE-A 2 944 850). In manchen Fällen läßt allerdings auch die Wirksamkeit dieser Stoffe zu wünschen übrig.

Ferner ist bekannt, daß zur Ethylenabspaltung befähigte Phosphonsäure-Derivate das Pflanzenwachstum beeinflussen (vgl. DE-A 2 053 967). Jedoch ist auch die Aktivität dieser Substanzen in der Praxis nicht immer ausreichend.

Schließlich wurde schon beschrieben, daß bestimmte 1-Amino-cyclopropan-1-carbonsäure-Derivate pflanzenwachstumsregulierende Eigenschaften aufweisen (vgl. DE-A 2 824 517). Die mit diesen Stoffen erzielten Ergebnisse sind aber ebenfalls nicht immer voll zufriedenstellend.

Im übrigen ist auch bekannt, daß Mischungen aus bestimmten Azol-Derivaten und 2-Chlorethanphosphonsäure pflanzenwuchsregulierende Wirksamkeit besitzen (vgl. EP-A 0 005 600 und Research Disclosure 176, 44-47 (1978)). Die betreffenden Azol-Derivate unterscheiden sich jedoch konstitutionell von den erfindungsgemäß verwendbaren Azolen. Außerdem werden keine pflanzenwuchsregulierend wirksamen Mischungen mit synergistischem Effekt erwähnt.

Es wurde nun gefunden, daß die neuen Wirkstoffkombinationen aus

A) einem Azol der Formel

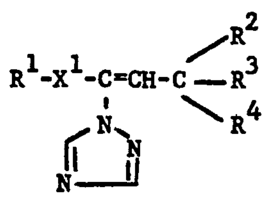

(I)

in welcher

$R^1$ für gegebenenfalls substituiertes Alkyl, Cycloalkyl oder gegebenenfalls substituiertes Aryl steht,

$R^2$ für Wasserstoff oder Alkyl steht,

$R^3$ für Wasserstoff, Alkyl, Cycloalkyl, gegebenenfalls substituiertes Cycloalkenyl, Alkenyl oder gegebenenfalls substituiertes Aryl steht,

$R^2$ und $R^3$ gemeinsam mit dem Kohlenstoffatom, an das sie gebunden sind, für gegebenenfalls substituiertes Cycloalkenyl oder Cycloalkyl stehen,

$R^4$ für Wasserstoff oder Alkyl steht,

$X^1$ für die Gruppe

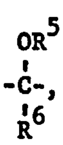

steht, sowie zusätzlich für die Ketogruppe steht, wenn $R^1$ für gegebenenfalls substituiertes Alkyl oder Cycloalkyl steht,

$R^5$ für Wasserstoff, Alkyl, gegebenenfalls substituiertes Aralkyl, Acyl oder gegebenenfalls substituiertes Carbamoyl steht und

$R^6$ für Wasserstoff, Alkyl oder gegebenenfalls substituiertes Aralkyl steht,

oder einem Azol der Formel

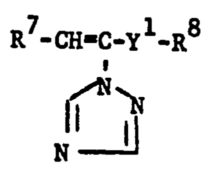

(II)

in welcher
$R^7$ für gegebenenfalls substituiertes Aryl steht,
$R^8$ für Alkyl, Halogenalkyl oder gegebenenfalls substituiertes Aryl steht und
$Y^1$ für Carbonyl oder für die Gruppe —CH(OH)— steht,
oder einem Azol-Derivat der Formel

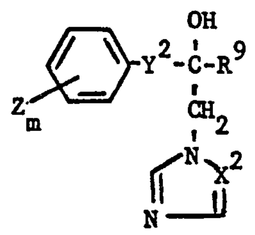
(III)

in welcher
$R^9$ für Alkyl, gegebenenfalls substituiertes Cycloalkyl oder gegebenenfalls substituiertes Phenyl steht,
$X^2$ für ein Stickstoffatom oder die CH-Gruppe steht,
$Y^2$ für die Gruppierungen $—OCH_2—$, $—CH_2CH_2—$ oder —CH=CH— steht, wobei im Falle der $—OCH_2$-Gruppe das Sauerstoffatom mit dem Phenylrest verbunden ist,
Z für Halogen, Alkyl, Cycloalkyl, Alkoxy, Alkylthio, Halogenalkyl, Halogenalkoxy, Halogenalkylthio, gegebenenfalls substituiertes Phenyl, gegebenenfalls substituiertes Phenoxy, gegebenenfalls substituiertes Phenylalkyl oder gegebenenfalls substituiertes Phenylalkoxy steht und
m für die Zahlen 0, 1, 2 oder 3 steht,
oder einem Säureadditions-Salz oder Metallsalz-Komplex von Azolen der Formeln (I), (II) oder (III), oder einem Pyrimidin-butanol-Derivat der Formel

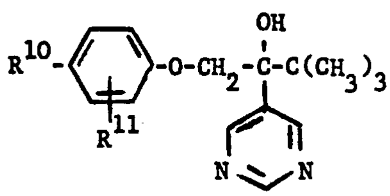
(IV)

in welcher
$R^{10}$ für Wasserstoff, Halogen, Alkyl, Alkoxy oder gegebenenfalls durch Halogen substituiertes Benzyloxy steht und
$R^{11}$ für Wasserstoff oder Halogen steht,
und
B) einem Phosphonsäure-Derivat der Formel

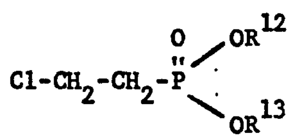
(V)

in welcher
$R^{12}$ und $R^{13}$ unabhängig voneinander für Wasserstoff oder ein Alkalimetall-Kation stehen,
oder einem 1-Amino-cyclopropan-1-carbonsäure-Derivat der Formel

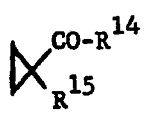
(VI)

in welcher
$R^{14}$ für Hydroxy, Alkoxy, Aralkoxy, Amino, Alkylamino, Dialkylamino oder für den Rest

$$O^{\ominus}M^{\oplus}$$

steht, wobei $M^{\oplus}$ für ein Alkali- oder Erdalkalimetallionenäquivalent oder für ein Ammonium-, Alkylammonium-, Dialkylammonium-, Trialkylammonium- oder Tetraalkylammoniumion steht,

und

$R^{15}$ für Amino oder für den Rest —NH—CO—R steht, worin R für Wasserstoff, Alkyl oder Aryl steht und

$R^{15}$ ferner für den Rest $—N^{\oplus}H_3$ $A^{\ominus}$ steht, wobei $A^{\ominus}$ für Chlorid, Bromid oder Jodid steht, sehr gut zur Hemmung des Pflanzenwachstums geeignet sind.

Überraschenderweise ist die pflanzenwuchshemmende Wirkung der erfindungsgemäßen Wirkstoffkombinationen wesentlich höher als die Summe der Wirkungen der einzelnen Wirkstoffe. Es liegt also ein nicht vorhersehbarer echter synergistischer Effekt vor und nicht nur eine Wirkungsergänzung. Die Wirkstoffkombinationen stellen somit eine wertvolle Bereicherung der Technik dar.

Die in den erfindungsgemäßen Wirkstoffkombinationen enthaltenen Azole sind durch die Formeln (I), (II) und (III) allgemein definiert.

In der Formel (I) steht $R^1$ vorzugsweise für gegebenenfalls einfach oder zweifach substituiertes, geradkettiges oder verzweigtes Alkyl mit 1 bis 4 Kohlenstoffatomen, wobei als Substituenten vorzugsweise in Frage kommen : Halogen, Alkylcarbonyloxy mit 1 bis 4 Kohlenstoffatomen im Alkylteil, Alkylsulfonyloxy mit 1 bis 4 Kohlenstoffatomen sowie gegebenenfalls durch Halogen oder Alkyl mit 1 bis 4 Kohlenstoffatomen substituiertes Phenylsulfonyloxy. $R^1$ steht außerdem vorzugsweise für Cycloalkyl mit 5 bis 7 Kohlenstoffatomen sowie für gegebenenfalls einfach oder mehrfach, gleich oder verschieden substituiertes Aryl mit 6 bis 10 Kohlenstoffatomen, wie Phenyl oder Naphthyl, wobei als Substituenten vorzugsweise in Frage kommen : Halogen, Alkyl mit 1 bis 4 Kohlenstoffatomen, Phenyl, Phenoxy, Halogenphenyl und Halogenphenoxy. $R^2$ steht vorzugsweise für Wasserstoff und geradkettiges oder verzweigtes Alkyl mit 1 bis 4 Kohlenstoffatomen. $R^3$ steht vorzugsweise für Wasserstoff, geradkettiges oder verzweigtes Alkyl mit 1 bis 4 Kohlenstoffatomen, Cycloalkyl mit 5 bis 7 Kohlenstoffatomen, gegebenenfalls durch Alkyl mit 1 bis 4 Kohlenstoffatomen substituiertes Cycloalkenyl mit 5 bis 7 Kohlenstoffatomen, Alkenyl mit 2 bis 4 Kohlenstoffatomen oder für gegebenenfalls substituiertes Aryl mit 6 bis 10 Kohlenstoffatomen, wie Phenyl und Naphthyl, wobei als Substituenten vorzugsweise Halogen und Alkyl mit 1 bis 4 Kohlenstoffatomen in Frage kommen. $R^2$ und $R^3$ stehen außerdem vorzugsweise gemeinsam mit dem Kohlenstoffatom, an das sie gebunden sind, für gegebenenfalls durch Alkyl mit 1 bis 4 Kohlenstoffatomen substituiertes Cycloalkenyl mit 5 bis 7 Kohlenstoffatomen und Cycloalkyl mit 3 bis 7 Kohlenstoffatomen. $R^4$ steht vorzugsweise für Wasserstoff oder Alkyl mit 1 bis 4 Kohlenstoffatomen. $X^1$ steht vorzugsweise für die Gruppe $—C(OR^5)R^6$ sowie zusätzlich für die Ketogruppe, wenn $R^1$ für gegebenenfalls substituiertes Alkyl oder Cycloalkyl steht. $R^5$ steht vorzugsweise für Wasserstoff, geradkettiges oder verzweigtes Alkyl mit 1 bis 4 Kohlenstoffatomen sowie für gegebenenfalls einfach oder mehrfach, gleich oder verschieden substituiertes Aralkyl mit 1 bis 2 Kohlenstoffatomen im Alkylteil und 6 bis 10 Kohlenstoffatomen im Arylteil, wie Benzyl oder Naphthylmethyl, wobei als Substituenten vorzugsweise in Frage kommen : Halogen, Alkyl mit 1 bis 4 Kohlenstoffatomen, Halogenalkyl mit bis zu 2 Kohlenstoffatomen und bis zu 3 gleichen oder verschiedenen Halogenatomen, wobei als Halogenatome vorzugsweise Fluor und Chlor in Betracht kommen, sowie gegebenenfalls durch Halogen substituiertes Phenyl und Phenoxy. $R^5$ steht weiterhin vorzugsweise für den Acylrest $—CO—R^{16}$ und den Carbamoylrest $—CO—NR^{17}R^{18}$. $R^6$ steht vorzugsweise für Wasserstoff, Alkyl mit 1 bis 4 Kohlenstoffatomen sowie für gegebenenfalls durch Halogen oder Alkyl mit 1 bis 4 Kohlenstoffatomen substituiertes Aralkyl mit 1 bis 2 Kohlenstoffatomen im Alkylteil und 6 bis 10 Kohlenstoffatomen im Arylteil, wie insbesondere Benzyl. $R^{16}$ steht vorzugsweise für geradkettiges oder verzweigtes Alkyl mit 1 bis 4 Kohlenstoffatomen, Halogenalkyl mit 1 bis 2 Kohlenstoffatomen und 1 bis 5 gleichen oder verschiedenen Halogenatomen, vorzugsweise Fluor- und Chloratomen, sowie für gegebenenfalls substituiertes Phenyl oder Benzyl, wobei als Substituenten vorzugsweise in Frage kommen : Halogen und Alkyl mit 1 bis 4 Kohlenstoffatomen. $R^{17}$ steht vorzugsweise für Wasserstoff oder Alkyl mit 1 bis 4 Kohlenstoffatomen. $R^{18}$ steht vorzugsweise für Alkyl mit 1 bis 8 Kohlenstoffatomen, Halogenalkyl mit bis zu 4 Kohlenstoff- und bis zu 5 gleichen oder verschiedenen Halogenatomen, wie insbesondere Fluor- und Chloratomen, gegebenenfalls einfach oder mehrfach, gleich oder verschieden substituiertes Aryl mit 6 bis 10 Kohlenstoffatomen, wie Phenyl und Naphthyl, wobei als Substituenten vorzugsweise in Frage kommen : Halogen, Alkyl mit 1 bis 4 Kohlenstoffatomen sowie Halogenalkyl mit bis zu 2 Kohlenstoff- und bis zu 5 gleichen oder verschiedenen Halogenatomen, wie insbesondere Fluor- und Chloratomen, sowie vorzugsweise für Halogenalkylmercapto mit 1 bis 2 Kohlenstoff- und bis zu 5 Halogenatomen, wie insbesondere Fluor- und Chloratomen.

Ganz besonders bevorzugt sind diejenigen Verbindungen der Formel (I), in denen $R^1$ für tert.-Butyl, Isopropyl, Chlor-tert.-butyl, Brom-tert.-butyl, Fluor-tert.-butyl, Acetoxy-tert.-butyl, Methylsulfonyloxy-tert.-butyl, p-Toluolsulfonyloxy-tert.-butyl ; 1,3-Dichlor-2-methyl-prop-2-yl ; 1,3-Dibrom-2-methyl-prop-2-yl ; 1,3-Difluor-2-methyl-prop-2-yl ; 1-Chlor-3-brom-2-methyl-prop-2-yl ; 1,3-Diacetoxy-2-methyl-prop-2-yl ; Cyclohexyl, Phenyl, Chlorphenyl, Bromphenyl, Dichlorphenyl, Fluorphenyl, Methylphenyl, Dimethylphenyl, Chlor-methylphenyl, Biphenyl, Phenoxyphenyl, Chlorphenylphenyl oder Chlorphenoxyphenyl steht ; $R^2$ für Wasserstoff, Methyl, Ethyl, Propyl oder Butyl steht ; $R^3$ für Wasserstoff, Methyl, Ethyl, Isopropyl, Cyclohexyl, Cyclohexenyl, Methylcyclohexenyl, Allyl, Methacryl, Phenyl, Chlorphenyl, Dichlorphenyl oder Methylphenyl steht ; $R^2$ und $R^3$ gemeinsam mit dem Kohlenstoffatom, an das sie gebunden sind, für Cyclopropyl, Cyclobutyl, Cyclopentyl, Cyclohexyl, Cyclohexenyl oder Methylcyclohexenyl stehen ; $R^4$ für Wasserstoff, Methyl, Ethyl oder n-Propyl steht, $X^1$ für die Gruppe $—C(OR^5)R^6—$ steht sowie auch für die

Ketogruppe steht, wenn $R^1$ für die angegebenen Bedeutungen als gegebenenfalls substituiertes Alkyl oder Cycloalkyl steht; $R^5$ für Wasserstoff, Methyl, Ethyl, n-Propyl, Isopropyl, Isobutyl, gegebenenfalls einfach oder mehrfach, gleich oder verschieden durch Chlor, Fluor, Methyl, Phenyl, Chlorphenyl, Phenoxy, Chlorphenoxy substituiertes Benzyl, den Acylrest $—COR^{16}$ oder den Carbamoylrest $—CO—NR^{17}R^{18}$ steht; $R^6$ für Wasserstoff, Methyl, Ethyl, Isopropyl, Benzyl, Chlorbenzyl oder Dichlorbenzyl steht; $R^{16}$ für Methyl, Ethyl, Isopropyl, Isobutyl, Chlormethyl, Dichlormethyl oder gegebenenfalls einfach oder mehrfach substituiertes Phenyl und Benzyl mit Chlor, Brom oder Methyl als Substituenten steht; $R^{17}$ für Wasserstoff, Methyl oder Ethyl steht und $R^{18}$ für Methyl, Ethyl, Chlorethyl, Phenyl, Chlorphenyl, Trifluormethyl-, Chlordifluor-methyl-, Dichlorfluor-methyl- oder Trichlormethylmercapto steht.

Im einzelnen seien außer den bei den Herstellungsbeispielen genannten Verbindungen folgende Verbindungen der allgemeinen Formel (II) genannt:

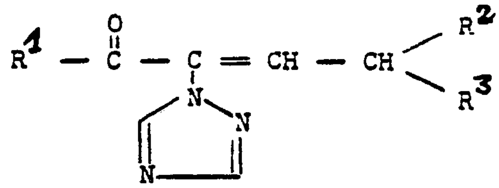

(Ia)

| $R^1$ | $R^2$ | $R^3$ |
|---|---|---|
| $C(CH_3)_3$ | $C_2H_5$ | $C_2H_5$ |
| $C(CH_3)_3$ | $C_2H_5$ | $CH_3$ |
| $C(CH_3)_3$ | $CH_3$ | $CH_3$ |
| $C(CH_3)_3$ | $CH_3$ | 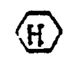 |
| $C(CH_3)_3$ | $CH_3$ | 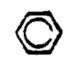 |
| $C(CH_3)_3$ | Cyclopropyl | |
| $C(CH_3)_3$ | Cyclobutyl | |
| $C(CH_3)_3$ | Cyclopentyl | |
| $C(CH_3)_3$ | Cycloheptyl | |
| $C(CH_3)_3$ | Norborn-3-en-2-yl | |
| 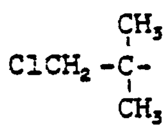 | Cyclohexyl | |
| 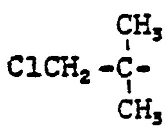 | $CH_3$ | $CH_3$ |
| 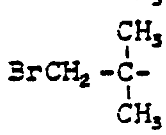 | Cyclohexyl | |
| 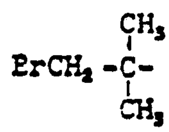 | $CH_3$ | $CH_3$ |
| 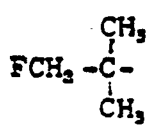 | Cyclohexyl | |
| 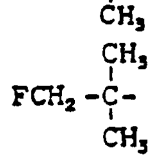 | $CH_3$ | $CH_3$ |

(Fortsetzung)

| $R^1$ | $R^2$ | $R^3$ |
|---|---|---|
| 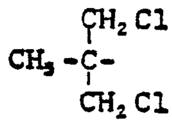 | Cyclohexyl | |
| 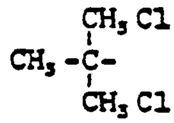 | $CH_3$ | $CH_3$ |
| 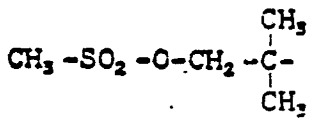 | Cyclohexyl | |
| 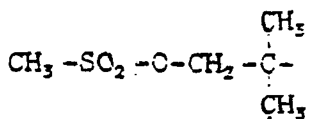 | $CH_3$ | $CH_3$ |
| 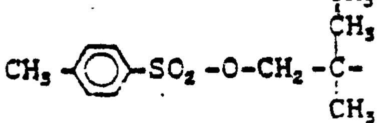 | Cyclohexyl | |
| 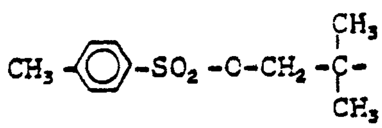 | $CH_3$ | $CH_3$ |
| 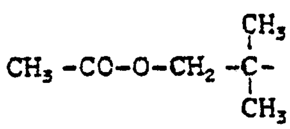 | Cyclohexyl | |
| 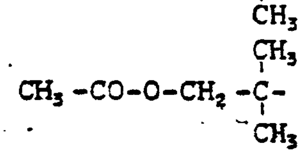 | $CH_3$ | $CH_3$ |
| 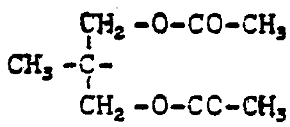 | Cyclohexyl | |
| 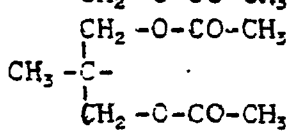 | $CH_3$ | $CH_3$ |
| 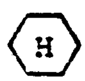 | Cyclohexyl | |
| 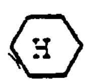 | $CH_3$ | $CH_3$ |

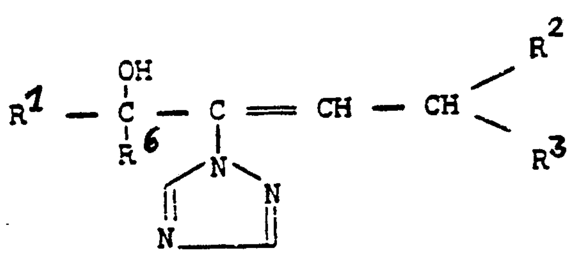 (Ib)

| $R^1$ | $R^2$ | $R^3$ | $R^6$ |
|---|---|---|---|
| $C(CH_3)_3$ | $C_2H_5$ | $CH_3$ | H |
| $C(CH_3)_3$ | $CH_3$ | $CH_3$ | H |
| $C(CH_3)_3$ | $CH_3$ | 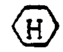 | H |
| $C(CH_3)_3$ | $CH_3$ | 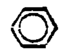 | H |
| $C(CH_3)_3$ | Cyclopropyl | | H |
| $C(CH_3)_3$ | Cyclobutyl | | H |
| $C(CH_3)_3$ | Cyclopentyl | | H |
| $C(CH_3)_3$ | Cycloheptyl | | H |
| $C(CH_3)_3$ | $CH_3$ | $CH_3$ | $CH_3$ |
| $C(CH_3)_3$ | Cyclohexyl | | $CH_3$ |
| $C(CH_3)_3$ | $CH_3$ | $CH_3$ | $-CH_2-$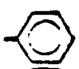 |
| $C(CH_3)_3$ | Cyclohexyl | | $-CH_2-$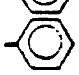 |
| 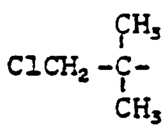 | $CH_3$ | $CH_3$ | H |
| 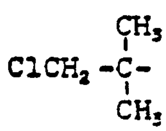 | Cyclohexyl | | H |
| 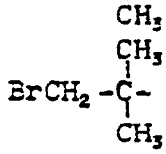 | $CH_3$ | $CH_3$ | H |
| 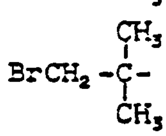 | Cyclohexyl | | H |
| 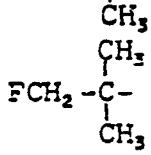 | $CH_3$ | $CH_3$ | H |
| 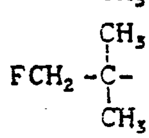 | Cyclohexyl | | H |
| 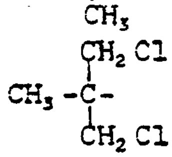 | $CH_3$ | $CH_3$ | H |
| 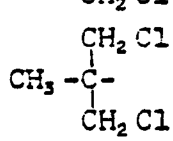 | Cyclohexyl | | H |
| 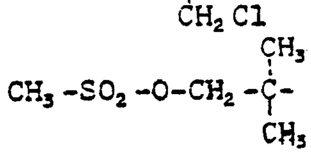 | $CH_3$ | $CH_3$ | H |

(Fortsetzung)

| $R^1$ | $R^2$ | $R^3$ | $R^6$ |
| --- | --- | --- | --- |
| 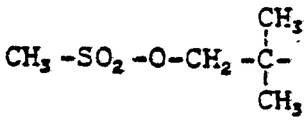 | Cyclohexyl | | H |
| 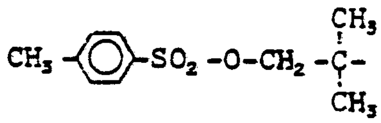 | $CH_3$ | $CH_3$ | H |
| 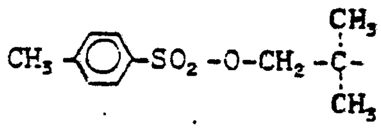 | Cyclohexyl | | H |
| 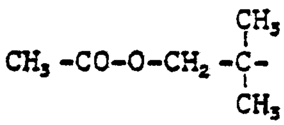 | $CH_3$ | $CH_3$ | H |
| 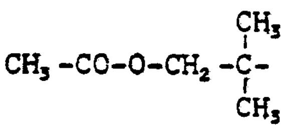 | Cyclohexyl | | H |
| 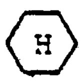 | $CH_3$ | $CH_3$ | H |
| 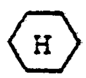 | Cyclohexyl | | H |
| 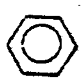 | $CH_3$ | $CH_3$ | H |
| 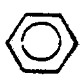 | Cyclohexyl | | H |
| 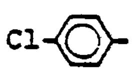 | $CH_3$ | $CH_3$ | H |
| 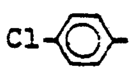 | Cyclohexyl | | H |
| 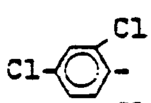 | $CH_3$ | $CH_3$ | H |
| 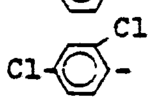 | Cyclohexyl | | H |

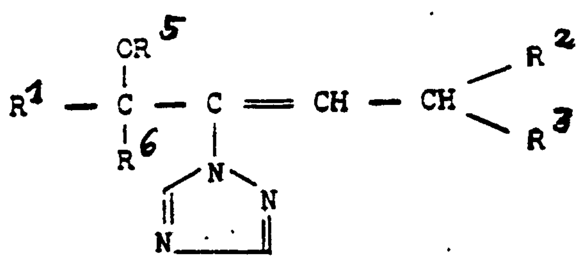

(Ic)

(Fortsetzung)

| $R^1$ | $R^2$ | $R^3$ | $R^5$ | $R^6$ |
|---|---|---|---|---|
| $C(CH_3)_3$ | $CH_3$ | $CH_3$ | $C_2H_5$ | H |
| $C(CH_3)_3$ | Cyclohexyl | | $C_2H_5$ | H |
| 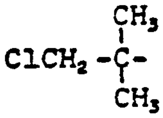 | $CH_3$ | $CH_3$ | $C_2H_5$ | $CH_3$ |
| 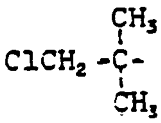 | Cyclohexyl | | $C_2H_5$ | $CH_3$ |
| 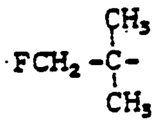 | $CH_3$ | $CH_3$ | $C_2H_5$ | H |
| 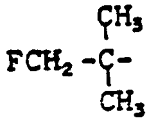 | Cyclohexyl | | $C_2H_5$ | H |
| 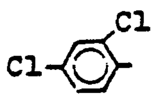 | $CH_3$ | $CH_3$ | $C_2H_5$ | H |
| 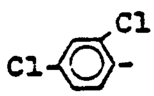 | Cyclohexyl | | $C_2H_5$ | H |
| $C(CH_3)_3$ | $CH_3$ | $CH_3$ | 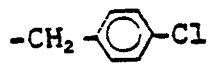 | H |
| $C(CH_3)_3$ | Cyclohexyl | | 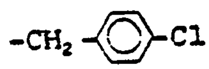 | H |
| 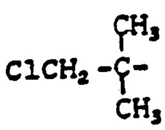 | $CH_3$ | $CH_3$ | 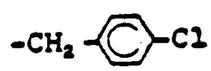 | H |
| 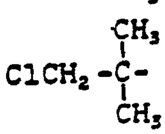 | Cyclohexyl | | 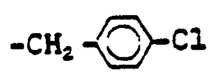 | H |
| 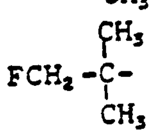 | $CH_3$ | $CH_3$ | 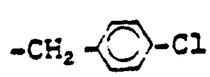 | H |
| 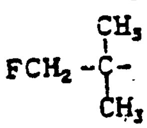 | Cyclohexyl | | 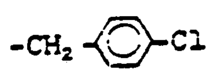 | H |
| 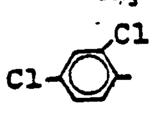 | $CH_3$ | $CH_3$ | 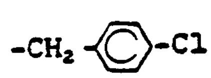 | H |
| 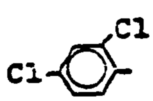 | Cyclohexyl | | 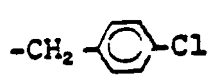 | H |

(Fortsetzung)

| $R^1$ | $R^2$ | $R^3$ | $R^5$ | $R^6$ |
|---|---|---|---|---|
| 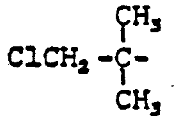 | $CH_3$ | $CH_3$ | $-CO-CH_3$ | H |
| 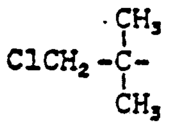 | Cyclohexyl | | $-CO-CH_3$ | H |
| 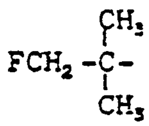 | $CH_3$ | $CH_3$ | $-CO-CH_3$ | H |
| 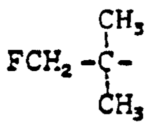 | Cyclohexyl | | $-CO-CH_3$ | H |
| 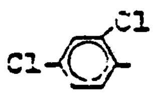 | $CH_3$ | $CH_3$ | $-CO-CH_3$ | H |
| 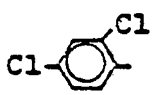 | Cyclohexyl | | $-CO-CH_3$ | H |
| 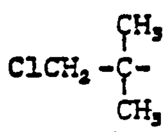 | $CH_3$ | $CH_3$ | $-CO-NHCH_3$ | H |
| 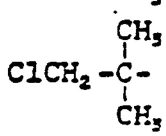 | Cyclohexyl | | $-CO-NHCH_3$ | H |
| 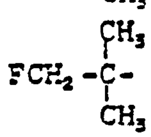 | $CH_3$ | $CH_3$ | $-CO-NHCH_3$ | H |
| 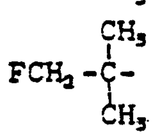 | Cyclohexyl | | $-CO-NHCH_3$ | H |
| 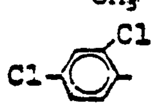 | $CH_3$ | $CH_3$ | $-CO-NHCH_3$ | H |
| 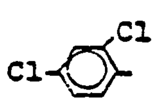 | Cyclohexyl | | $-CO-NHCH_3$ | H |
| 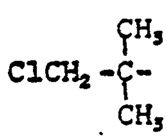 | $CH_3$ | $CH_3$ | -CO-NH- 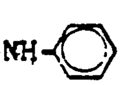 | H |
| 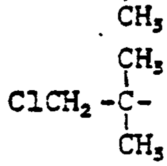 | Cyclohexyl | | -CO-NH- 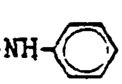 | H |

(Fortsetzung)

| R¹ | R² | R³ | R⁵ | R⁶ |
|---|---|---|---|---|
| 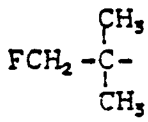 | $CH_3$ | $CH_3$ | 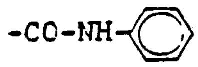 | H |
| 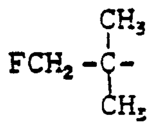 | Cyclohexyl | | 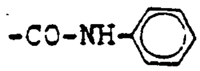 | H |
| 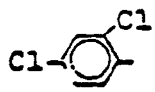 | $CH_3$ | $CH_3$ | 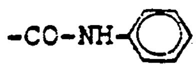 | H |
| 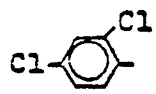 | Cyclohexyl | | 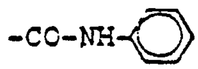 | H |
| 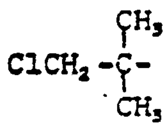 | $CH_3$ | $CH_3$ | 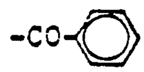 | H |
| 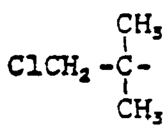 | Cyclohexyl | | 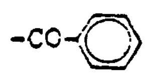 | H |
| 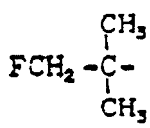 | $CH_3$ | $CH_3$ | 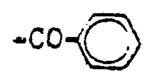 | H |
| 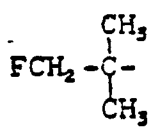 | Cyclohexyl | | 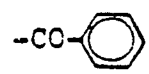 | H |
| 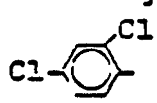 | $CH_3$ | $CH_3$ | 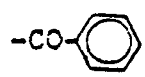 | H |
| 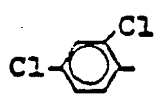 | Cyclohexyl | | 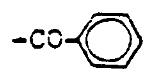 | H |
| 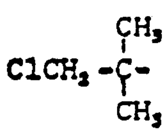 | $CH_3$ | $CH_3$ | $-CO-CHCl_2$ | H |
| 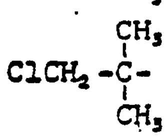 | Cyclohexyl | | $-CO-CHCl_2$ | H |
| 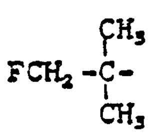 | $CH_3$ | $CH_3$ | $-CO-CHCl_2$ | H |
| 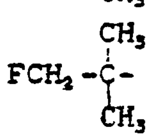 | Cyclohexyl | | $-CO-CHCl_2$ | H |

(Fortsetzung)

| $R^1$ | $R^2$ | $R^3$ | $R^5$ | $R^6$ |
|---|---|---|---|---|
| 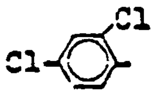 | $CH_3$ | $CH_3$ | $-CO-CHCl_2$ | H |
| 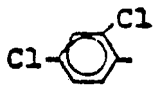 | Cyclohexyl | | $-CO-CHCl_2$ | H |
| $C(CH_3)_3$ | $CH_3$ | $CH_3$ | $-CO-CH_3$ | H |
| $C(CH_3)_3$ | Cyclohexyl | | $-CO-CH_3$ | H |
| $C(CH_3)_3$ | $CH_3$ | $CH_3$ | $-CO-NHCH_3$ | H |
| $C(CH_3)_3$ | Cyclohexyl | | $-CO-NHCH_3$ | H |
| $C(CH_3)_3$ | $CH_3$ | $CH_3$ | -CO-NH-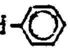 | H |
| $C(CH_3)_3$ | Cyclohexyl | | -CO-NH-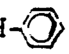 | H |
| $C(CH_3)_3$ | $CH_3$ | $CH_3$ | -CO-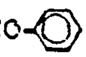 | H |
| $C(CH_3)_3$ | Cyclohexyl | | -CO-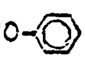 | H |
| $C(CH_3)_3$ | $CH_3$ | $CH_3$ | $-CO-CHCl_2$ | H |
| $C(CH_3)_3$ | Cyclohexyl | | $-CO-CHCl_2$ | H |

Weitere besonders bevorzugte Azole der Formel (I) sind in den Herstellungsbeispielen genannt.

Die Azole der Formel (I) sind bereits bekannt (vgl. DE-A 2 906 061 und DE-A 2 645 617).

Die Verbindungen der Formel (I) können in zwei geometrischen Isomerenformen vorliegen, je nach Anordnung der Gruppen, die an die Doppelbindung gebunden sind. Wenn $X^1$ für die Gruppe $—C(OR^5)R^6—$ steht, liegt ein asymmetrisches Kohlenstoffatom vor, so daß die Verbindungen der Formel (I) in diesem Fall außerdem in zwei optischen Isomerenformen anfallen. — Die vorliegende Erfindung betrifft sowohl die Verwendung der einzelnen Isomeren als auch die Verwendung der Isomeren-Gemische.

In der Formel (II) steht $R^7$ vorzugsweise für Phenyl, welches einfach, zweifach oder dreifach gleichartig oder verschieden substituiert sein kann durch Fluor, Chlor, Brom, Phenyl, Phenoxy, Nitro, Alkyl mit 1 bis 4 Kohlenstoffatomen, Alkoxy mit 1 bis 4 Kohlenstoffatomen, Cycloalkyl mit 3 bis 7 Kohlenstoffatomen, Halogenalkyl mit 1 bis 4 Kohlenstoffatomen und 1 bis 5 Halogenatomen, insbesondere Fluor-, Chlor- und Bromatomen, und/oder durch Cyano. $R^8$ steht vorzugsweise für geradkettiges oder verzweigtes Alkyl mit 1 bis 6 Kohlenstoffatomen, für Halogenalkyl mit 1 bis 6 Kohlenstoffatomen und 1 bis 5 Halogenatomen, wobei als Halogenatome vorzugsweise Fluor, Chlor und Brom genannt seien. $R^8$ steht ferner vorzugsweise für Phenyl, welches einfach, zweifach oder dreifach gleichartig oder verschieden substituiert sein kann durch Fluor, Chlor, Brom, Phenyl, Phenoxy, Nitro, Alkyl mit 1 bis 4 Kohlenstoffatomen, Alkoxy mit 1 bis 4 Kohlenstoffatomen, Cycloalkyl mit 3 bis 7 Kohlenstoffatomen, Halogenalkyl mit 1 bis 4 Kohlenstoffatomen und 1 bis 5 Halogenatomen, insbesondere Fluor-, Chlor- und Bromatomen, und/oder durch Cyano. $Y^1$ steht für Carbonyl oder die Gruppe —CH(OH)—.

Als Beispiele für Azole der Formel (II) seien im einzelnen die in der folgenden Tabelle aufgeführten Verbindungen genannt :

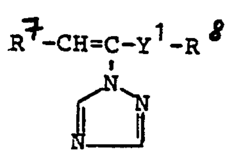

(II)

(Siehe Tabelle, Seite 13 f.)

| $R^7$ | $Y^1$ | $R^8$ |
|---|---|---|
| 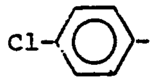 | CH(OH) | tert.-$C_4H_9$ |
| 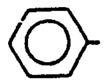 | CH(OH) | tert.-$C_4H_9$ |
| 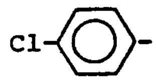 | CH(OH) | $-C(CH_3)_2CH_2Cl$ |
| 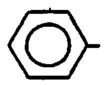 | CO | tert.-$C_4H_9$ |
| 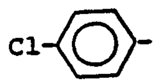 | CO | tert.-$C_4H_9$ |
| 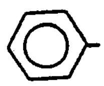 | CO | $-C(CH_3)_2CH_2Cl$ |
| 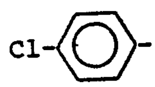 | CO | $-C(CH_3)_2CH_2Cl$ |
| 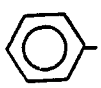 | CO | 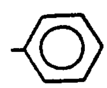 |
| 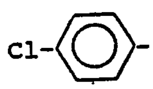 | CO | 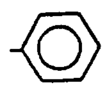 |
| 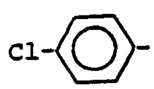 | CO | 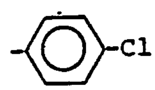 |
| 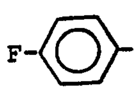 | CO | 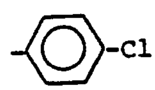 |
| 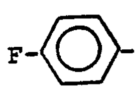 | CO | 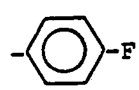 |
| 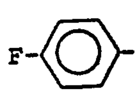 | CO | 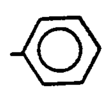 |
| 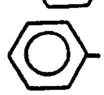 | CO | $-CH_3$ |
| 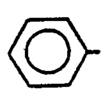 | CO | $-C_2H_5$ |
| 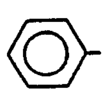 | CO, | $-C_3H_7$-n |

(Fortsetzung)

| R[7] | $Y^1$ | $R^8$ |
|---|---|---|
| 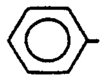 | CO | $-C_3H_7$-iso |
| 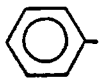 | CH(OH) | tert.-$C_4H_9$ |
| 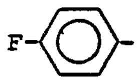 | CH(OH) | tert.-$C_4H_9$ |
| 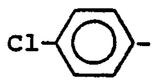 | CH(OH) | tert.-$C_4H_9$ |
| 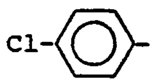 | CO | $-C(CH_2Cl)_2CH_3$ |
| 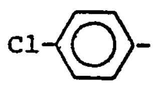 | CH(OH) | $-C(CH_2Cl)_2CH_3$ |
| 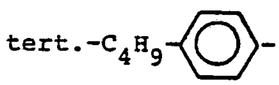 | CO | $-C(CH_2Cl)_2CH_3$ |
| 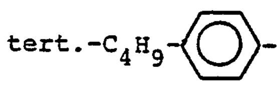 | CH(OH) | $-C(CH_2Cl)_2CH_3$ |
| 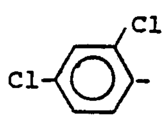 | CO | $-C(CH_2Cl)_2CH_3$ |
| 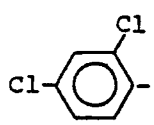 | CH(OH) | $-C(CH_2Cl)_2CH_3$ |
| 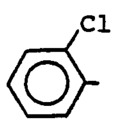 | CO | $-C(CH_2Cl)_2CH_3$ |
| 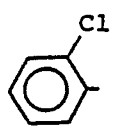 | CH(OH) | $-C(CH_2Cl)_2CH_3$ |
| 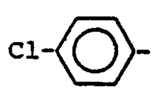 | CO | $-C(CH_2F)_2CH_3$ |
| 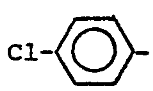 | CH(OH) | $-C(CH_2F)_2CH_3$ |
| 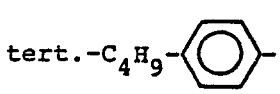 | CO | $-C(CH_2F)_2CH_3$ |

(Fortsetzung)

| $R^7$ | $Y^1$ | $R^8$ |
|---|---|---|
| 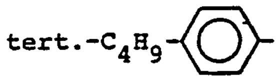 | CH(OH) | $-C(CH_2F)_2CH_3$ |
| 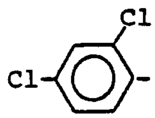 | CO | $-C(CH_2F)_2CH_3$ |
| 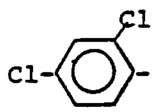 | CH(OH) | $-C(CH_2F)_2CH_3$ |
| 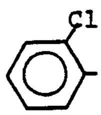 | CO | $-C(CH_2F)_2CH_3$ |
| 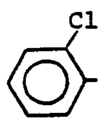 | CH(OH) | $-C(CH_2F)_2CH_3$ |
| 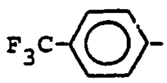 | CO | $-C(CH_3)_2CH_2Cl$ |
| 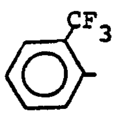 | CO | $-C(CH_3)_2CH_2Cl$ |
| 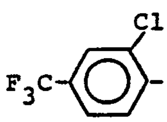 | CO | $-C(CH_3)_2CH_2Cl$ |
| 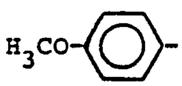 | CO | $-C(CH_3)_2CH_2Cl$ |
| 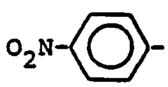 | CO | $-C(CH_3)_2CH_2Cl$ |
| 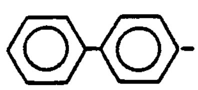 | CO | $-C(CH_3)_2CH_2Cl$ |
| 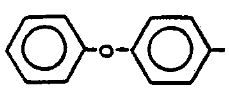 | CO | $-C(CH_3)_2CH_2Cl$ |
| 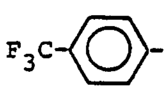 | CH(OH) | $-C(CH_3)_2CH_2Cl$ |
| 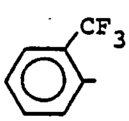 | CH(OH) | $-C(CH_3)_2CH_2Cl$ |
| 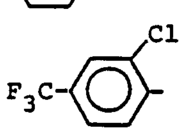 | CH(OH) | $-C(CH_3)_2CH_2Cl$ |

(Fortsetzung)

| $R^7$ | $Y^1$ | $R^8$ |
|---|---|---|
| 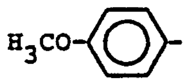 | CH(OH) | $-C(CH_3)_2CH_2Cl$ |
| 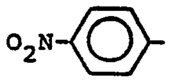 | CH(OH) | $-C(CH_3)_2CH_2Cl$ |
| 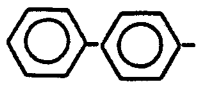 | CH(OH) | $-C(CH_3)_2CH_2Cl$ |
| 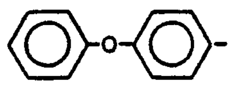 | CH(OH) | $-C(CH_3)_2CH_2Cl$ |
| 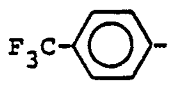 | CO | $-C(CH_3)_2CH_2F$ |
| 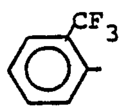 | CO | $-C(CH_3)_2CH_2F$ |
| 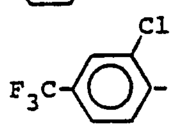 | CO | $-C(CH_3)_2CH_2F$ |
| 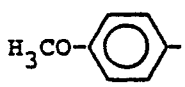 | CO | $-C(CH_3)_2CH_2F$ |
| 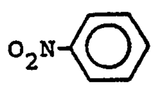 | CO | $-C(CH_3)_2CH_2F$ |
| 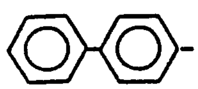 | CO | $-C(CH_3)_2CH_2F$ |
| 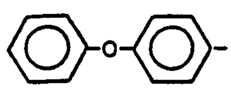 | CO | $-C(CH_3)_2CH_2F$ |
| 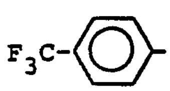 | CH(OH) | $-C(CH_3)_2CH_2F$ |
| 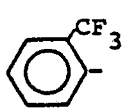 | CH(OH) | $-C(CH_3)_2CH_2F$ |
| 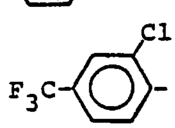 | CH(OH) | $-C(CH_3)_2CH_2F$ |
| 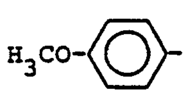 | CH(OH) | $-C(CH_3)_2CH_2F$ |

(Fortsetzung)

| $R^7$ | $Y^1$ | $R^8$ |
|---|---|---|
| 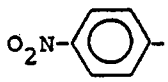 | CH(OH) | $-C(CH_3)_2CH_2F$ |
| 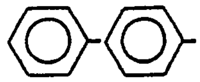 | CH(OH) | $-C(CH_3)_2CH_2F$ |
| 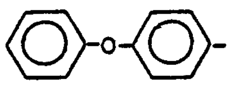 | CH(OH) | $-C(CH_3)_2CH_2F$ |

Weitere besonders bevorzugte Azole der Formel (II) sind in den Herstellungsbeispielen genannt.

Die Azole der Formel (II) sind bekannt (vgl. DE-A 2 645 617, DE-A 2 838 847, JP-A 53 130 661, DE-A 2 920 437 und DE-A 30 25 242).

Die Azole der Formel (II) können in 2 geometrischen Isomerenformen vorliegen, je nach Anordnung der Gruppen, die an die Doppelbindung gebunden sind. Wenn $Y^1$ für die Gruppe —CH(OH)— steht, liegt ein asymmetrisches Kohlenstoffatom vor, so daß die Verbindungen der Formel (II) in dem Fall auch in 2 optischen Isomerenformen anfallen. Die vorliegende Erfindung betrifft sowohl die Verwendung der einzelnen Isomeren als auch die Verwendung der Isomeren-Gemische.

In der Formel (III) steht $R^9$ vorzugsweise für geradkettiges oder verzweigtes Alkyl mit 1 bis 4 Kohlenstoffatomen, gegebenenfalls durch Alkyl mit 1 bis 2 Kohlenstoffatomen substituiertes Cycloalkyl mit 3 bis 7 Kohlenstoffatomen sowie für gegebenenfalls einfach oder mehrfach, gleich oder verschieden substituiertes Phenyl, wobei als Substituenten vorzugsweise in Frage kommen : Halogen, Alkyl mit 1 bis 4 Kohlenstoffatomen sowie Halogenalkyl mit 1 bis 2 Kohlenstoff- und 1 bis 5 gleichen oder verschiedenen Halogenatomen, wie insbesondere Fluor- und Chloratomen. Z steht vorzugsweise für Halogen, geradkettiges oder verzweigtes Alkyl mit 1 bis 4 Kohlenstoffatomen, Cycloalkyl mit 5 bis 7 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen, wie insbesondere Fluor- und Chloratomen sowie für jeweils gegebenenfalls substituiertes Phenyl, Phenoxy und Phenylalkyl sowie Phenylalkoxy mit 1 bis 2 Kohlenstoffatomen im Alkylteil bzw. im Alkoxyteil, wobei als Substituenten genannt seien : Halogen und Alkyl mit 1 bis 4 Kohlenstoffatomen. $X^2$, $Y^2$ und der Index m haben die oben angegebene Bedeutung.

Besonders bevorzugt sind diejenigen Verbindungen der Formel (III), in denen $R^9$ für tert.-Butyl, Isopropyl oder Methyl steht, für jeweils gegebenenfalls durch Methyl substituiertes Cyclopropyl, Cyclopentyl oder Cyclohexyl steht sowie für gegebenenfalls einfach oder zweifach, gleich oder verschieden durch Fluor, Chlor, Methyl oder Trifluormethyl substituiertes Phenyl steht ; Z für Fluor, Chlor, Brom, Methyl, tert.-Butyl, Cyclohexyl, Methoxy, Methylthio, Trifluormethyl, Trifluormethoxy, Trifluormethylthio sowie für jeweils gegebenenfalls einfach oder zweifach, gleich oder verschieden durch Fluor, Chlor und/oder Methyl substituiertes Phenyl, Phenoxy, Benzyl oder Benzyloxy steht, und $X^2$, $Y^2$ sowie der Index m die in der Erfindungsdefinition angegebene Bedeutung haben.

Im einzelnen seien außer den bei den Herstellungsbeispielen genannten Verbindungen folgende Verbindungen der allgemeinen Formel (III) genannt :

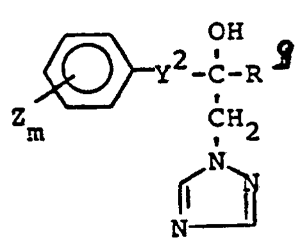

(III)

(Siehe Tabelle, Seite 18 ff.)

| $Z_m$ | $Y^2$ | $R^9$ | $X^2$ |
|---|---|---|---|
| 4-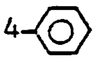 | $-O-CH_2-$ | $-C(CH_3)_3$ | N(CH) |
| 4-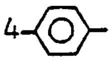- Cl | " | " | " |
| 4-O-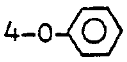 | " | " | " |
| 4-O-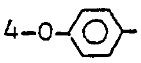- Cl | " | " | " |
| $4-CH_2-$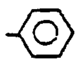 | " | " | " |
| $4-CH_2-$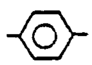- Cl | " | " | " |
| $4-O-CH_2-$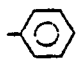 | " | " | " |
| $4-O-CH_2-$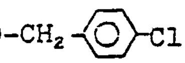-Cl | " | " | " |
| $3,4-Cl_2$ | " | " | " |
| $4-CF_3$ | " | " | " |
| $4-OCF_3$ | " | " | " |
| $4-SCF_3$ | " | " | " |
| $4-SCH_3$ | " | " | " |
| $4-C(CH_3)_3$ | " | " | " |
| 4-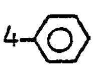 | $-O-CH_2$ | 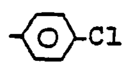-Cl | N (CH) |
| 4-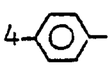- Cl | " | " | " |
| 4-O-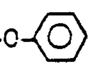 | " | " | " |
| 4-O-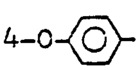- Cl | " | " | " |
| $4-CH_2-$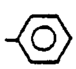 | " | " | " |
| $4-CH_2-$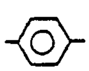- Cl | " | " | " |
| $4-O-CH_2-$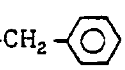 | " | " | " |
| $4-O-CH_2-$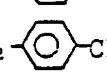-Cl | " | " | " |
| $3,4-Cl_2$ | " | " | " |
| $4-CF_3$ | " | " | " |
| $4-OCF_3$ | " | " | " |
| $4-SCF_3$ | " | " | " |
| $4-SCH_3$ | " | " | " |

(Fortsetzung)

| $Z_m$ | $Y^2$ | $R^9$ | $X^2$ |
|---|---|---|---|
| $4-C(CH_3)_3$ | " | " | " |
| 4- 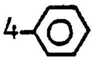 | $-O-CH_2-$ | $-CH(CH_3)_2$ | N (CH) |
| 4- 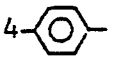 Cl | " | " | " |
| 4-O- 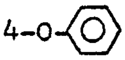 | " | " | " |
| 4-O- 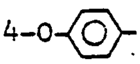 Cl | " | " | " |
| $4-CH_2-$ 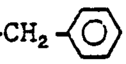 | " | " | " |
| $4-CH_2-$ 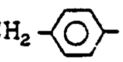 Cl | " | " | " |
| $4-O-CH_2-$ 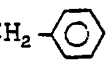 | " | " | " |
| $4-O-CH_2-$ 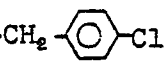 Cl | " | " | " |
| $3,4-Cl_2$ | " | " | " |
| $4-CF_3$ | " | " | " |
| $4-OCF_3$ | " | " | " |
| $4-SCF_3$ | " | " | " |
| $4-SCH_3$ | " | " | " |
| $4-C(CH_3)_3$ | " | " | " |
| 4- 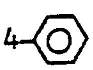 | $-O-CH_2-$ | 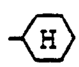 | N (CH) |
| 4- 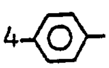 Cl | " | " | " |
| 4-O- 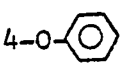 | " | " | " |
| 4-O- 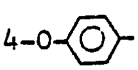 Cl | " | " | " |
| $4-CH_2-$ 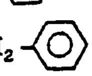 | " | " | " |
| $4-CH_2-$ 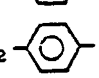 Cl | " | " | " |
| $4-O-CH_2-$ 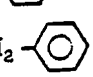 | " | " | " |
| $4-O-CH_2-$ 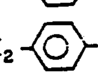 Cl | " | " | " |
| $3,4-Cl_2$ | " | " | " |
| $4-CF_3$ | " | " | " |
| $4-OCF_3$ | " | " | " |
| $4-SCF_3$ | " | " | " |

(Fortsetzung)

| $Z_m$ | $Y^2$ | $R^9$ | $X^2$ |
|---|---|---|---|
| 4-$SCH_3$ | " | " | " |
| 4-$C(CH_3)_3$ | " | " | " |
| 4-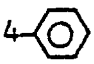 | -O-$CH_2$- | 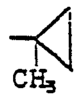 | N(CH) |
| 4-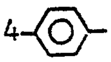Cl | " | " | " |
| 4-O-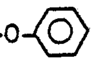 | " | " | " |
| 4-O-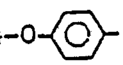Cl | " | " | " |
| 4-$CH_2$-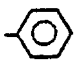 | " | " | " |
| 4-$CH_2$-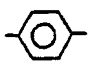Cl | " | " | " |
| 4-O-$CH_2$-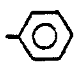 | " | " | " |
| 4-O-$CH_2$-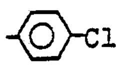Cl | " | " | " |
| 3,4-$Cl_2$ | " | " | " |
| 4-$CF_3$ | " | " | " |
| 4-$OCF_3$ | " | " | " |
| 4-$SCF_3$ | " | " | " |
| 4-$SCH_3$ | " | " | " |
| 4-$C(CH_3)_3$ | " | " | " |
| 4-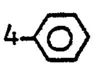 | -$CH_2$-$CH_2$- | -$C(CH_3)_3$ | N(CH) |
| 4-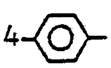Cl | " | " | " |
| 4-O-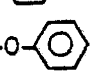 | " | " | " |
| 4-O-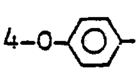Cl | " | " | " |
| 4-$CH_2$-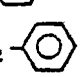 | " | " | " |
| 4-$CH_2$-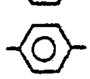Cl | " | " | " |
| 4-O-$CH_2$-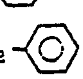 | " | " | " |
| 4-O-$CH_2$-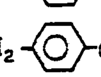Cl | " | " | " |
| 3,4-$Cl_2$ | " | " | " |
| 4-$CF_3$ | " | " | " |

(Fortsetzung)

| $Z_m$ | $Y^2$ | $R^9$ | $X^2$ |
|---|---|---|---|
| 4-$OCF_3$ | " | " | " |
| 4-$SCF_3$ | " | " | " |
| 4-$SCH_3$ | " | " | " |
| 4-$C(CH_3)_3$ | " | " | " |
| 4-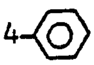 | $-CH_2-CH_2-$ | 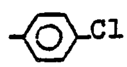Cl | N(CH) |
| 4-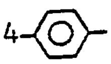 Cl | " | " | " |
| 4-O-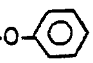 | " | " | " |
| 4-O-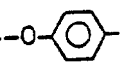 Cl | " | " | " |
| 4-$CH_2$-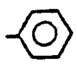 | " | " | " |
| 4-$CH_2$-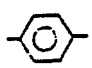 Cl | " | " | " |
| 4-O-$CH_2$-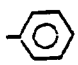 | " | " | " |
| 4-O-$CH_2$-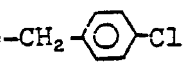Cl | " | " | " |
| 3,4-$Cl_2$ | " | " | " |
| 4-$CF_3$ | " | " | " |
| 4-$OCF_3$ | " | " | " |
| 4-$SCF_3$ | " | " | " |
| 4-$SCH_3$ | " | " | " |
| 4-$C(CH_3)_3$ | " | " | " |
| 4-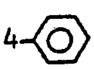 | $-CH_2-CH_2-$ | $-CH(CH_3)_2$ | N(CH) |
| 4-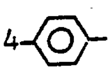 Cl | " | " | " |
| 4-O-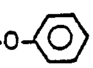 | " | " | " |
| 4-O-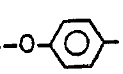 Cl | " | " | " |
| 4-$CH_2$-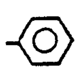 | " | " | " |
| 4-$CH_2$-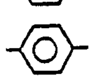 Cl | " | " | " |
| 4-O-$CH_2$-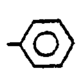 | " | " | " |
| 4-O-$CH_2$-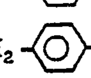Cl | " | " | " |

(Fortsetzung)

| $Z_m$ | $Y^2$ | $R^9$ | $X^2$ |
|---|---|---|---|
| 3,4-$Cl_2$ | " | " | " |
| 4-$CF_3$ | " | " | " |
| 4-$OCF_3$ | " | " | " |
| 4-$SCF_3$ | " | " | " |
| 4-$SCH_3$ | " | " | " |
| 4-$C(CH_3)_3$ | " | " | " |
| 4-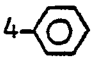 | $-CH_2-CH_2-$ | 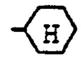 | N (CH) |
| 4-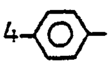 Cl | " | " | " |
| 4-O-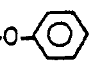 | " | " | " |
| 4-O-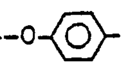 Cl | " | " | " |
| 4-$CH_2$-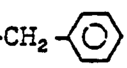 | " | " | " |
| 4-$CH_2$-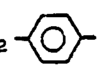 Cl | " | " | " |
| 4-O-$CH_2$-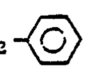 | " | " | " |
| 4-O-$CH_2$-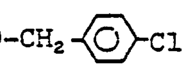Cl | " | " | " |
| 3,4-$Cl_2$ | " | " | " |
| 4-$CF_3$ | " | " | " |
| 4-$OCF_3$ | " | " | " |
| 4-$SCF_3$ | " | " | " |
| 4-$SCH_3$ | " | " | " |
| 4-$C(CH_3)_3$ | " | " | " |
| 4-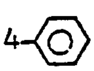 | $-CH_2-CH_2-$ | 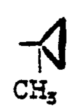 | N(CH) |
| 4-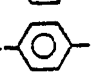 Cl | " | " | " |
| 4-O-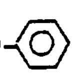 | " | " | " |
| 4-O-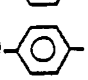 Cl | " | " | " |
| 4-$CH_2$-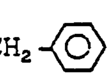 | " | " | " |
| 4-$CH_2$-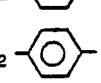 Cl | " | " | " |

(Fortsetzung)

| $Z_m$ | $Y^2$ | $R^9$ | $X^2$ |
|---|---|---|---|
| $4-O-CH_2-$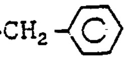 | " | " | " |
| $4-O-CH_2-$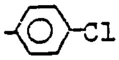$-Cl$ | " | " | " |
| $3,4-Cl_2$ | " | " | " |
| $4-CF_3$ | " | " | " |
| $4-OCF_3$ | " | " | " |
| $4-SCF_3$ | " | " | " |
| $4-SCH_3$ | " | " | " |
| $4-C(CH_3)_3$ | " | " | " |
| 4-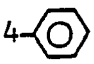 | -CH=CH- | $-C(CH_3)_3$ | N(CH) |
| 4-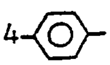- Cl | " | " | " |
| 4-O-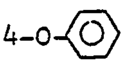 | " | " | " |
| 4-O-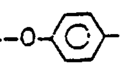- Cl | " | " | " |
| $4-CH_2-$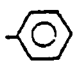 | " | " | " |
| $4-CH_2-$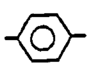- Cl | " | " | " |
| $4-O-CH_2-$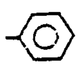 | " | " | " |
| $4-O-CH_2-$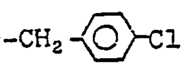$-Cl$ | " | " | " |
| $3,4-Cl_2$ | " | " | " |
| $4-CF_3$ | " | " | " |
| $4-OCF_3$ | " | " | " |
| $4-SCF_3$ | " | " | " |
| $4-SCH_3$ | " | " | " |
| $4-C(CH_3)_3$ | " | " | " |
| 4-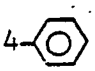 | -CH=CH- | 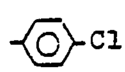-Cl | N(CH) |
| 4-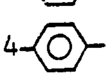- Cl | " | " | " |
| 4-O-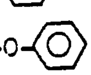 | " | " | " |
| 4-O-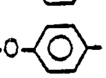- Cl | " | " | " |

(Fortsetzung)

| $Z_m$ | $Y^2$ | $R^9$ | $X^2$ |
|---|---|---|---|
| $4-CH_2-$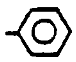 | " | " | - " |
| $4-CH_2-$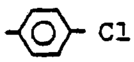 | " | " | " |
| $4-O-CH_2-$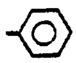 | " | " | " |
| $4-O-CH_2-$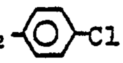 | " | " | " |
| $3,4-Cl_2$ | " | " | " |
| $4-CF_3$ | " | " | " |
| $4-OCF_3$ | " | " | " |
| $4-SCF_3$ | " | " | " |
| $4-SCH_3$ | " | " | " |
| $4-C(CH_3)_3$ | " | " | " |
| 4-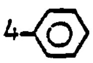 | $-CH=CH-$ | $-CH(CH_3)_2$ | N(CH) |
| 4-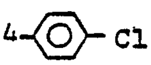 | " | " | " |
| 4-O-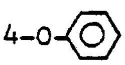 | " | " | " |
| 4-O-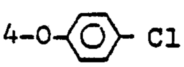 | " | " | " |
| $4-CH_2-$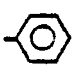 | " | " | - " |
| $4-CH_2-$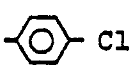 | " | " | " |
| $4-O-CH_2-$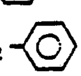 | " | " | " |
| $4-O-CH_2-$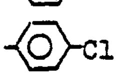 | " | " | " |
| $3,4-Cl_2$ | " | " | " |
| $4-CF_3$ | " | " | " |
| $4-OCF_3$ | " | " | " |
| $4-SCF_3$ | " | " | " |
| $4-SCH_3$ | " | " | " |
| $4-C(CH_3)_3$ | " | " | " |
| 4-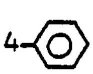 | $-CH=CH-$ | 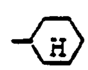 | N(CH) |
| 4-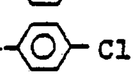 | " | " | " |
| 4-O-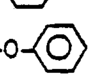 | " | " | " |

(Fortsetzung)

| $Z_m$ | $Y^2$ | $R^9$ | $X^2$ |
|---|---|---|---|
| 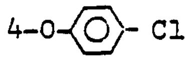 | " | " | " |
| 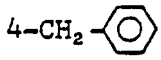 | " | " | " |
| 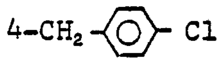 | " | " | " |
| 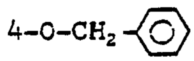 | " | " | " |
| 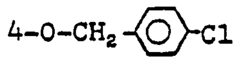 | " | " | " |
| 3,4-$Cl_2$ | " | " | " |
| 4-$CF_3$ | " | " | " |
| 4-$OCF_3$ | " | " | " |
| 4-$SCF_3$ | " | " | " |
| 4-$SCH_3$ | " | " | " |
| 4-$C(CH_3)_3$ | " | " | " |
| 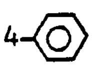 | -CH=CH- | 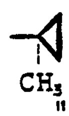 | N (CH) |
| 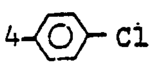 | " | " | " |
| 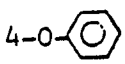 | " | " | " |
| 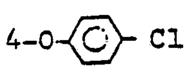 | " | " | " |
| 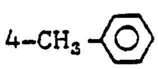 | " | " | " |
| 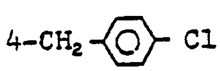 | " | " | " |
| 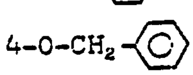 | " | " | " |
| 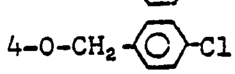 | " | " | " |
| 3,4-$Cl_2$ | " | " | " |
| 4-$CF_3$ | " | " | " |
| 4-$OCF_3$ | " | " | " |
| 4-$SCF_3$ | " | " | " |
| 4-$SCH_3$ | " | " | " |
| 4-$C(CH_3)_3$ | " | " | " |
| 4-Cl | $-O-CH_2-$ | $-CH(CH_3)_2$ | N(CH) |
| 4-F | " | " | " |

(Fortsetzung)

| $Z_m$ | $Y^2$ | $R^9$ | $X^2$ |
|---|---|---|---|
| 4-$CH_3$ | " | " | " |
| 4-Cl | $-O-CH_2-$ | 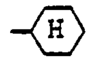 | N (CH) |
| 4-F | " | " | " |
| 4-$CH_3$ | " | " | " |
| 4-Cl | $-O-CH_2-$ | 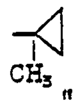 | N (CH) |
| 4-F | " | " | " |
| 4-$CH_3$ | " | " | " |
| 4-Cl | $-CH_2-CH_2-$ | $-CH(CH_3)_2$ | N (CH) |
| 4-F | " | " | " |
| 4-$CH_3$ | " | " | " |
| 4-Cl | $-CH_2-CH_2-$ | 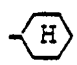 | N(CH) |
| 4-F | " | " | " |
| 4-$CH_3$ | " | " | " |
| 4-Cl | $-CH_2-CH_2-$ | 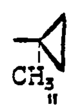 | N(CH) |
| 4-F | " | " | " |
| 4-$CH_3$ | " | " | " |
| 4-Cl | $-CH=CH-$ | $-CH(CH_3)_2$ | N(CH) |
| 4-F | " | " | " |
| 4-$CH_3$ | " | " | " |
| 4-Cl | $-CH=CH-$ | 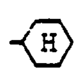 | N (CH) |
| 4-F | " | " | " |
| 4-$CH_3$ | " | " | " |

(Fortsetzung)

| $Z_m$ | $Y^2$ | $R^9$ | $X^2$ |
|---|---|---|---|
| 4-Cl | -CH=CH- | 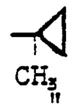 | N(CH) |
| 4-F | " | " | " |
| $4-CH_3$ | " | " | " |
| $2,4-Cl_2$ | $-CH_2-CH_2-$ | $-C(CH_3)_3$ | N(CH) |
| $4-CH_3$ | " | " | " |
| $4-Cl, 2-CH_3$ | " | " | " |
| $2,4-Cl_2$ | -CH=CH- | $-C(CH_3)_3$ | N(CH) |
| $4-CH_3$ | " | " | " |
| $4-Cl, 2-CH_3$ | " | " | " |
| 4-F | $-O-CH_2-$ | $-C(CH_3)_3$ | N(CH) |
| $2-CH_3$ | $-CH_2-CH_2-$ | $-C(CH_3)_3$ | N |
| 4-F | -CH=CH- | $-C(CH_3)_3$ | N |

Weitere besonders bevorzugte Azole der Formel (III) sind in den Herstellungsbeispielen genannt.

Die Azole der Formel (III) sind bekannt (vgl. EP-A 40 345).

In der Formel (IV) steht $R^{10}$ vorzugsweise für Wasserstoff, Fluor, Chlor, Brom, Alkyl mit 1 bis 4 Kohlenstoffatomen oder gegebenenfalls durch Chlor substituiertes Benzyloxy. $R^{11}$ steht vorzugsweise für Wasserstoff, Fluor, Chlor oder Brom.

Ganz besonders bevorzugt sind diejenigen Verbindungen der Formel (IV), in denen $R^{10}$ für Wasserstoff, Fluor, Chlor, Methyl, Ethyl, Isopropyl, Methoxy, Ethoxy, Isopropoxy, Benzyloxy oder Chlorbenzyloxy steht und $R^{11}$ für Wasserstoff, Chlor oder Fluor steht.

Weitere besonders bevorzugte Pyrimidin-butanol-Derivate der Formel (IV) sind in den Herstellungsbeispielen angegeben.

Die Pyrimidin-butanol-Derivate der Formel (IV) sind bereits bekannt (vgl. DE-A 2 742 173).

Zur Herstellung von Säureadditionssalzen von Azolen der Formeln (I), (II) und (III) kommen vorzugsweise folgende Säuren in Frage : Die Halogenwasserstoffsäuren, wie z. B. Chlorwasserstoffsäure und die Bromwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salicylsäure, Sorbinsäure, Milchsäure sowie Sulfonsäuren, wie z. B. p-Toluolsulfonsäure und 1,5-Naphthalindisulfonsäure.

Die Säureadditions-Salze der Verbindungen der Formeln (I) bis (III) können in einfacher Weise nach üblichen Salzbildungsmethoden, z. B. durch Lösung einer Verbindung der Formeln (I) bis (III) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formeln (I) bis (III) kommen

vorzugsweise Salze von Metallen der II. bis IV. Hauptgruppe und der I. und II. sowie IV. bis VIII. Nebengruppe des Periodensystems der Elemente in Frage, wobei Kupfer, Zink, Mangan, Magnesium, Zinn, Eisen und Nickel beispielhaft genannt seien.

Als Anionen der Salze kommen solche in Betracht, die sich vorzugsweise von folgenden Säuren ableiten : Halogenwasserstoffsäuren, wie z. B. die Chlorwasserstoffsäure und die Bromwasserstoffsäure, ferner Phosphorsäure, Salpetersäure und Schwefelsäure.

Die Metall-Komplexe von Verbindungen der Formeln (I) bis (III) können in einfacher Weise nach üblichen Verfahren erhalten werden, so z. B. durch Lösen des Metallsalzes in Alkohol, z. B. Ethanol und Hinzufügen zu einer Verbindung der Formeln (I) bis (III). Man kann Metallsalz-Komplexe in bekannter Weise, z. B. durch Anfiltrieren, Isolieren und gegebenenfalls durch Umkristallisieren reinigen.

In der Formel (V) stehen $R^{12}$ und $R^{13}$ vorzugsweise für Wasserstoff oder ein Natrium-Kation.

Die Phosphonsäure-Derivate der Formel (V) sind bereits bekannt (vgl. DE-A 2 053 967).

In der Formel (VI) stehen vorzugsweise

$R^{14}$ für Hydroxy, Alkoxy mit 1 bis 20 Kohlenstoffatomen, Benzyloxy, Amino, Alkylamino mit 1 bis 4 Kohlenstoffatomen, Dialkylamino mit 1 bis 4 Kohlenstoffatomen je Alkylrest oder für den Rest

$O^{\ominus}M^{\oplus}$

wobei $M^{\oplus}$ für ein Natrium- oder Kaliumion, ein Magnesium- oder Calciumionenäquivalent, Ammonium, Alkylammonium mit 1 bis 4 Kohlenstoffatomen, Dialkylammonium, Trialkylammonium oder Tetraalkylammonium mit jeweils 1 bis 4 Kohlenstoffatomen je Alkylrest steht,

$R^{15}$ für Amino oder den Rest —NH—CO—R, worin R für Wasserstoff, Alkyl mit 1 bis 4 Kohlenstoffatomen oder Phenyl steht,

oder für den Rest $—N^{\oplus}H_3Cl^{\ominus}$.

Ganz besonders bevorzugt sind solche Verbindungen der Formel (VI), in denen

$R^{14}$ für Hydroxy, Alkoxy mit 1 bis 10 Kohlenstoffatomen, Benzyloxy, Amino, Alkylamino mit 1 oder 2 Kohlenstoffatomen, Dialkylamino mit 1 oder 2 Kohlenstoffatomen je Alkylrest oder für den Rest

$O^{\ominus}M^{\oplus}$

steht, wobei $M^{\oplus}$ für ein Natrium- oder Kaliumion, ein Magnesium- oder Calciumionenäquivalent, Ammonium, Alkylammonium mit 1 oder 2 Kohlenstoffatomen, Dialkylammonium, Trialkylammonium oder Tetraalkylammonium mit jeweils 1 oder 2 Kohlenstoffatomen je Alkylrest steht,

$R^{15}$ für Amino, Formylamino, Acetylamino, Propionylamino oder für $N^{\oplus}H_3Cl^{\ominus}$ steht.

Weitere besonders bevorzugte 1-Amino-cyclopropan-1-carbonsäure-Derivate der Formel (VI) sind in den Herstellungsbeispielen genannt.

Die 1-Amino-cyclopropan-1-carbonsäure-Derivate der Formel (VI) sind bereits bekannt (vgl. DE-A 2 824 517).

Wenn die Wirkstoffe in den erfindungsgemäßen Wirkstoffkombinationen in bestimmten Verhältnissen vorliegen, zeigt sich der synergistische Effekt besonders deutlich. Jedoch können die Gewichtsverhältnisse der Wirkstoffe in den Wirkstoffkombinationen in relativ großen Bereichen schwanken. Im allgemeinen entfallen auf 1 Gewichtsteil an einem unter A) aufgeführten Wirkstoff 0,05 bis 5 Gewichtsteile, vorzugsweise 0,1 bis 4 Gewichtsteile an einem der unter B) aufgeführten Wirkstoffe.

Die erfindungsgemäßen Wirkstoffkombinationen zeigen eine starke pflanzenwuchshemmende Wirksamkeit, insbesondere bei monokotylen Pflanzen wie z. B. Getreide und Gras.

Eine Hemmung des Längenwachstums bei Getreide ist deshalb von Bedeutung, weil dadurch auch unter ungünstigen Witterungsverhältnissen ein Umknicken der Halme (« Lagern ») des Getreides weitgehend vermieden wird. Durch Anwendung der erfindungsgemäßen Wirkstoffkombinationen ist es ferner möglich, eine Düngung mit größeren Mengen Stickstoffgünger auszubringen ohne Gefahr zu laufen, daß das Getreide lagert. Es lassen sich so durch die Anwendung der erfindungsgemäßen Wirkstoffkombinationen in Verbindung mit der Ausbringung einer größeren Düngermenge Mehrerträge erzielen.

Auch bei der Graswuchshemmung läßt sich der beschriebene synergistische Effekt der erfindungsgemäßen Wirkstoffkombinationen vorteilhaf nutzen. So kann z. B. die Häufigkeit der Grasschnitte in Ziergärten, Parkund Sportanlagen oder an Straßenrändern reduziert werden.

Unter Getreide sind im vorliegenden Fall alle üblichen Getreidearten zu verstehen. Hierzu gehören vorzugsweise Hafer, Roggen, Gerste, Weizen und Reis.

Die Wirkstoffkombinationen können in die üblichen Formulierungen übergeführt werden, wie Lösungen, Emulsionen, Suspensionen, Pulver und Granulate.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage : Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage :

Z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate ; als feste Trägerstoffe für Granulate kommen in Frage : z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel ; als Emulgiermittel kommen in Frage :

Z. B. nichtionogene und anionische Emulgatoren, wie Polyoxyethylen-Fettsäure-Ester, Polyoxyethylen-Fettalkohol-Ether, z. B. Alkylarylpolyglykol-ether, Alkylsulfonate, Alkylsulfate, Arylsulfonate sowie Eiweißhydrolysate ; als Dispergiermittel kommen in Frage : z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffkombinationen können in den Formulierungen in Mischung mit anderen bekannten Wirkstoffen vorliegen, wie Fungizide, Insektizide, Akarizide und Herbizide, sowie in Mischungen mit Düngemitteln.

Die Wirkstoffkombinationen können als solche, in Form ihrer Formulierungen oder der daraus bereiteten Anwendungsformen, wie gebrauchsfertige Lösungen, emulgierbare Konzentrate, Emulsionen, Suspensionen, Spritzpulver, lösliche Pulver und Granulate, angewendet werden.

Die Anwendung geschieht in üblicher Weise, z. B. durch Gießen, Verspritzen, Versprühen, Verstreuen, usw.

Die Wirkstoffkonzentrationen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wirkstoffkombinationen in einem bevorzugten Zeitraum vorgenommen wird, dessen genaue Abgrenzung sich nach den klimatischen und vegetativen Gegebenheiten richtet.

Die gute pflanzenwuchshemmende Wirkung der erfindungsgemäßen Wirkstoffkombinationen geht aus den nachfolgenden Beispielen hervor. Während die einzelnen Wirkstoffe in der wuchshemmenden Wirkung Schwächen aufweisen, zeigen die Kombinationen eine Wirkung, die über eine einfache Wirkungssummierung hinausgeht.

Ein synergistischer Effekt liegt bei Wuchshemmern immer dann vor, wenn die wuchshemmende Wirkung der Wirkstoffkombinationen größer ist als die Summe der Wirkungen der einzelnen applizierten Wirkstoffe.

Die zu erwartende Wirkung für eine gegebene Kombination zweier Wuchshemmer kann (vgl. Colby, S. R., « Calculating synergistic and antagonistic responses of herbicide combinations », Weeds 15, Seiten 20-22, 1967) wie folgt berechnet werden :

Wenn X = % Wuchshemmung durch Wirkstoff A bei p kg/ha Aufwandmenge,

und Y = % Wuchshemmung durch Wirkstoff B bei q kg/ha Aufwandmenge,

und E = die erwartete Wuchshemmung durch die Wirkstoffe A und B bei p und q kg/ha Aufwandmenge,

dann ist $E = X + Y - ((X \cdot Y)/100)$.

Ist die tatsächliche Wuchshemmung größer als berechnet, so ist die Kombination in ihrer Wirkung überadditiv, d. h. es liegt ein synergistischer Effekt vor.

Aus den Tabellen der Beispiele A und B geht eindeutig hervor, daß die gefundene wuchshemmende Wirkung der erfindungsgemäßen Wirkstoffkombinationen größer ist als die berechnete, d. h., daß ein synergistischer Effekt vorliegt.

In den folgenden Beispielen werden die nachstehend angegebenen Wirkstoffe eingesetzt :

(I-2) =

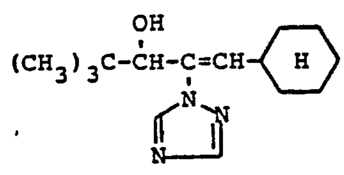

(II-1) = 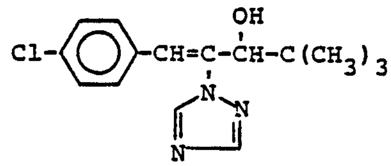

(II-4) = 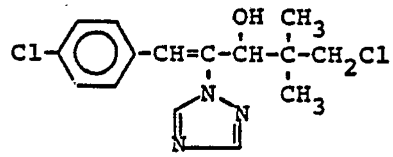

(III-1) = 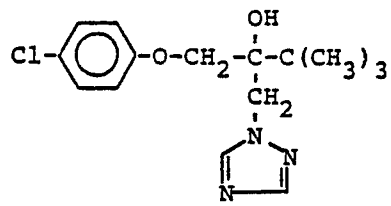

(IV-4) = 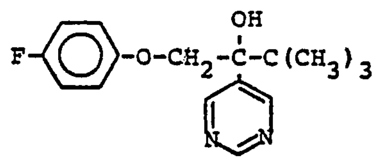

(V -1) = 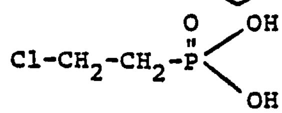

(VI -17) = 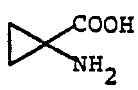

Beispiel A

Wuchshemmung bei Gerste

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil der Wirkstoffe mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen mit umgerechnet 500 l/ha der Wirkstoffzubereitungen besprüht. Nach 11 Tagen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Wirkstoffe, Aufwandmengen und Testergebnisse gehen aus der folgenden Tabelle hervor.

Tabelle A : Wuchshemmung bei Gerste

| Wirkstoffe bzw. Kombinationen | Wirkstoff-aufwandmenge kg/ha | Wuchshemmung in % gef.* | ber* |
|---|---|---|---|
| (VI -17) | 1 | 13 | - |
| (V -1) | 1 | 12 | - |

(Fortsetzung)

| Wirkstoffe bzw. Kombinationen | Wirkstoff-aufwandmenge kg/ha | Wuchshemmung in % gef.* | ber* |
|---|---|---|---|
| (III-1) | 2 | 19 | - |
| (III-1) + (V —1) | 2 + 1 | 50 | 28,7 |
| (II-4) | 2 | 8 | - |
| (II-4) + (VI —17) | 2 + 1 | 31 | 20,0 |
| (I-2) | 2 | 17 | - |
| (I-2) + (VI —17) | 2 + 1 | 35 | 27,8 |
| (I-2) + (V —1) | 2 + 1 | 37 | 27,0 |

*gef. = gefundene Wuchshemmung
*ber. = nach der oben angegebenen Formel berechnete Wuchshemmung

Beispiel B

Wuchshemmung bei Gerste

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil der Wirkstoffe mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen mit umgerechnet 500 l/ha der Wirkstoffzubereitungen besprüht. Nach 24 Tagen wird bei allen Pflanzen der Zuwachs gemessen und die Wuchshemmung in Prozent des Zuwaches der Kontrollpflanzen berechnet. Es bedeuten 100 % Wuchshemmung den Stillstand des Wachstums und 0 % ein Wachstum entsprechend dem der Kontrollpflanzen.

Wirkstoffe, Aufwandmengen und Testergebnisse gehen aus der folgenden Tabelle hervor.

Tabelle B : Wuchshemmung bei Gerste

| Wirkstoffe bzw. Kombinationen | Wirkstoff-aufwandmenge kg/ha | Wuchshemmung in % gef.* | ber.* |
|---|---|---|---|
| (VI -17) | 1 | 3 | - |
| (V -1) | 1 | 2 | - |
| (IV-4) | 2 | 25 | - |
| (IV-4) + (VI —17) | 2 + 1 | 31 | 27,3 |
| (III-1) | 2 | 9 | - |
| (III-1) + (V —1) | 2 + 1 | 22 | 10,8 |

(Fortsetzung)

| Wirkstoffe bzw. Kombinationen | Wirkstoffaufwandmenge kg/ha | Wuchshemmung in % gef.* | ber.* |
|---|---|---|---|
| (II-1) | 2 | 22 | - |
| (II-1) + (VI —17) | 2 + 1 | 33 | 24,3 |
| (I-2) | 2 | 5 | - |
| (I-2) + (VI —17) | 2 + 1 | 33 | 7,9 |
| (I-2) + (V —1) | 2 + 1 | 26 | 6,9 |

* gef. = gefundene Wuchshemmung
* ber. = nach der oben angegebenen Formel berechnete Wuchshemmung

Beispiel C

Wuchshemmung bei Gerste

Wäßrige Lösungen, die jeweils 1 Gew.-% Wirkstoff enthalten, werden hergestellt, indem man die jeweils benötigte Menge an der in den Beispielen 1 bis 3 beschriebenen Formulierungen mit Wasser vermischt. Diese wäßrigen Lösungen werden in dem jeweils gewünschten Verhältnis gemischt und mit Wasser auf die gewünschte Konzentration verdünnt.

Gerstepflanzen (Sorte « Gerda ») werden in Neubauer-Töpfen (8 Pflanzen pro Topf) im Freiland angezogen. Im 2-Knoten-Stadium werden die Pflanzen mit umgerechnet 500 l/ha der Wirkstoffzubereitungen besprüht. Nach 2 Monaten wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Wirkstoffe, Aufwandmengen und Testergebnisse gehen aus der folgenden Tabelle hervor.

Tabelle C : Wuchshemmung bei Gerste

| Wirkstoffe bzw. Kombinationen | Wirkstoffaufwandmenge kg/ha | Wuchshemmung in % gef.* | ber.* |
|---|---|---|---|
| (I-2) | 0,25 | 5 | - |
| | 0,5 | 9 | - |
| (VI —3) | 0,5 | 8 | - |
| (I-2) + (VI —3) | 0,25 + 0,5 | 21 | 12,6 |
| | 0,5 + 0,5 | 24 | 16,28 |
| (I-2) | 0,125 | 0 | - |
| (VI —22) | 0,5 | 12 | - |
| (I-2) + (VI —22) | 0,125 + 0,5 | 19 | 12 |

* gef. = gefundene Wuchshemmung
* ber. = nach der oben angegebenen Formel berechnete Wuchshemmung

Formulierungsbeispiele

Beispiel 1

Zur Herstellung eines Spritzpulvers werden

70 Gewichtsteile Wirkstoff gemäß Beispiel (II-2),
2 Gewichtsteile oberflächenaktives Mittel,
3 Gewichtsteile Dispergiermittel auf Basis von Ligninsulfonat,
5 Gewichtsteile Kondensationsprodukt aus Cyclohexanon, Formaldehyd und Natriumbisulfit,
5 Gewichtsteile hochdisperse Kieselsäure und
15 Gewichtsteile Kaolin

werden in einem Lödige-Mischer intensiv gemischt und anschließend in einem Mikronizer 8" fein gemahlen.

Beispiel 2

Zur Herstellung eines emulgierbaren Konzentrates werden

19,6 Gewichtsteile Wirkstoff gemäß Beispiel (VIII-3),
5,0 Gewichtsteile Alkylarylpolykolether und
75,4 Gewichtsteile Dimethylformamid

intensiv vermischt.

Beispiel 3

Zur Herstellung eines emulgierbaren Konzentrates werden

30,6 Gewichtsteile Wirkstoff gemäß Beispiel (VIII-22),
10,0 Gewichtsteile oberflächenaktives Mittel,
5,0 Gewichtsteile Alkylarylpolyglykolether und
54,4 Gewichtsteile Cyclohexanon

intensiv vermischt.

Herstellungsbeispiele

Beispiel (I-1)

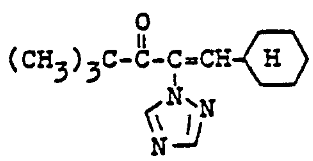

83,5 g (0,5 Mol) Pinakolyl-1,2,4-triazol, 60 g (0,54 Mol) Cyclohexanaldehyd, 4,2 g (0,05 Mol) Piperidin und 6 g (0,1 Mol) Eisessig in 300 ml Toluol werden am Wasserabscheider unter Rückfluß erhitzt, bis kein Wasser mehr übergeht. Nach dem Abkühlen der Reaktionslösung wird mit gesättigter Natriumchlorid-Lösung gewaschen, die organische Phase über Natriumsulfat getrocknet, filtriert und eingeengt. Der Rückstand wird in 500 ml Aceton aufgenommen und unter Rühren mit einer filtrierten Lösung von 90 g (0,25 Mol) Naphthalin-1,5-disulfonsäure in 500 ml Aceton versetzt.

Der zunächst ausfallende Niederschlag wird abgesaugt, das Filtrat weiter eingeengt und der erhaltene farblose kristalline Rückstand in 500 ml Methylenchlorid aufgenommen. Danach wird halbkonzentrierte Natriumcarbonat-Lösung bis zur alkalischen Reaktion zugegeben. Die organische Phase wird abgetrennt, getrocknet, filtriert und eingeengt. Der ölige Rückstand wird in Petrolether aufgenommen und der Kristallisation überlassen. Man erhält 64 g (49 % der Theorie) 1-Cyclohexyl-4,4-dimethyl-2-(1,2,4-triazol-1-yl)-pent-1-en-3-on vom Schmelzpunkt 98 °C.

Beispiel (I-2)

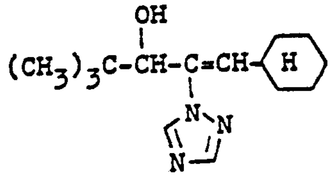

26 g (0,1 Mol) 1-Cyclohexyl-4,4-dimethyl-2-(1,2,4-triazol-1-yl)-pent-1-en-3-on (Beispiel II-1) werden in 200 ml Methanol aufgenommen und unter Rühren und Kühlen portionsweise mit 4,5 g Natriumborhydrid versetzt. Nach beendeter Reaktion wird das Reaktionsgemisch auf pH-6 eingestellt und eingeengt. Der Rückstand wird in 200 ml Methylenchlorid aufgenommen, mit gesättigter Natriumhydrogencarbonat-Lösung gewaschen, über Natriumsulfat getrocknet, filtriert und eingeengt. Der Rückstand wird aus Petrolether umkristallisiert. Man erhält 14,5 g (55 % der Theorie) 1-Cyclohexyl-4,4-dimethyl-2-(1,2,4-triazol-1-yl)-pent-1-en-3-ol vom Schmelzpunkt 131 °C.

Beispiel (I-3)

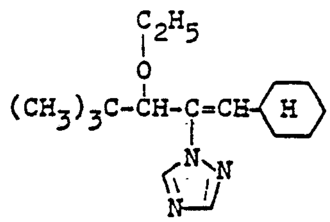

Eine Lösung von 26,3 g (0,1 mol) 1-Cyclohexyl-4,4-dimethyl-2-(1,2,4-triazol-1-yl)-pent-1-en-3-ol (Beispiel 2) in 50 ml Dioxan wird zu einer Suspension von 3 g 80 %-igem Natriumhydrid in 100 ml Dioxan getropft. Das Gemisch wird nach beendeter Zugabe 1 Stunde auf 50 °C erwärmt. Nach dem abkühlen werden 10,9 g (0,1 mol) Ethylbromid zugetropft und das Reaktionsgemisch über Nacht unter Rückfluß erhitzt. Nach dem Abkühlen werden 10 ml Methanol zugegeben und das Gemisch am Rotationsverdampfer eingeengt. Der Rückstand wird in Methylenchlorid aufgenommen und mit Wasser gewaschen. Nach dem Trocknen der organischen Phase über Natriumsulfat wird filtriert und das Filtrat eingeengt. Der Rückstand wird destilliert. Man erhält 11,0 g (37,8 % der Theorie) 1-Cyclohexyl-3-ethoxy-4,4-dimethyl-2-(1,2,4-triazol-1-yl)-pent-1-en vom Siedepunkt 110 °C/0,09 mbar.

Beispiel (I-4)

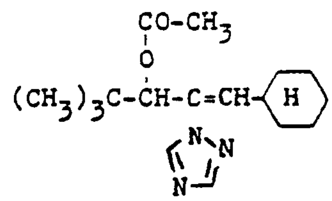

Eine Lösung von 13,15 g (0,05 mol)1-Cyclohexyl-4,4-dimethyl-2-(1,2,4-triazol-1-yl)-pent-1-en-3-ol (Beispiel 2) in 50 ml Dioxan wird zu einer Suspension von 1,5 g 80 %-igem Natriumhydrid in 50 ml Dioxan getropft. Nach beendeter Wasserstoffentwicklung werden 3,9 g (0,05 mol) Acetylchlorid zugetropft. Das Gemisch wird 4 Stunden unter Rückfluß erhitzt. Das Lösungsmittel wird nach dem Abkühlen im Vakuum abdestilliert, der Rückstand in Methylenchlorid aufgenommen und mit Wasser extrahiert. Die organische Phase wird über Natriumsulfat getrocknet, filtriert und die Lösung eingegeengt. Der Rückstand wird über eine Säule (Kieselgel ; Methanol : Chloroform = 1 : 3) gereinigt. Man erhält 5,6 g (35,4 % der Theorie) 3-Acetoxy-1-cyclohexyl-4,4-dimethyl-2-(1,2,4-triazol-1-yl)-pent-1-en als schwach gelbes Öl.

Zu einer Lösung von 13,15 g (0,05 Mol) 1-Cyclohexyl-4,4-dimethyl-2-(1,2,4-triazol-1-yl)-pent-1-en-3-ol (Beispiel II-2) in 100 ml Acetanhydrid werden 2 ml Pyridin gegeben. Das Gemisch wird vier Stunden bei 70 °C gerührt. Danach wird das Reaktionsgemisch auf Wasser gegossen und mit Natriumhydrogencarbonat neutralisiert. Die wäßrige Phase wird mehrmals mit Ether extrahiert. Die vereinigten Etherphasen werden über Natriumsulfat getrocknet und eingeengt. Man erhält 11,2 g (70,8 % der Theorie) 3-Acetoxy-1-cyclohexyl-4,4-dimethyl.-2-(1,2,4-triazol-1-yl)-pent-1-en als schwach gelbes Öl.

Beispiel (I-5)

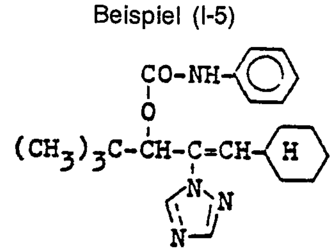

Zu einer Lösung von 13,15 g (0,05 mol) 1-Cyclohexyl-4,4-dimethyl-2-(1,2,4-triazol-1-yl)-pent-1-en-3-ol

(Beispiel II-2) in 100 ml Ether werden 6,5 g (0,055 mol) Phenylisocyanat und drei tropfen Tributylzinnlaurat als Katalysator gegeben. Das Gemisch wird 5 Tage bei Raumtemperatur gerührt. Nach Abdestillieren des Lösungsmittels im Vakuum wird der Rückstand aus Essigester/Ligroin umkristallisiert. Man erhält 4,8 g (25,1 % der Theorie) 1-Cyclohexyl-4,4-dimethyl-3-phenylcarbamoyloxy-2-(1,2,4-triazol-1-yl)-pent-1-en vom Schmelzpunkt 156 °C.

In analoger Weise werden die nachfolgenden Verbindungen 1 erhalten.

Tabelle 1

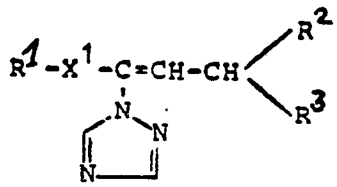

(Id)

| Bsp. Nr. | $R^1$ | $X^1$ | $R^2$ | $R^3$ | Schmelzpunkt (°C) |
|---|---|---|---|---|---|
| I-6 | $(CH_3)_3C$ | -CO- | 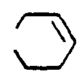 | | 193 (x ½ NDS) |
| I-7 | $(CH_3)_3C$ | -CO- | 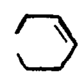 | | 40-48 |
| I-8 | $(CH_3)_3C$ | -CO- | 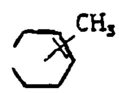 | | 49 |
| I-9 | $(CH_3)_3C$ | -CO- | 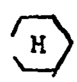 | | 201 (x ½ NDS) |
| I-10 | $(CH_3)_3C$ | -CO- | $n-C_4H_9$ | $C_2H_5$ | Öl |
| I-11 | $(CH_3)_3C$ | -CH(OH)- | 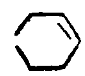 | | 151 (Z-Form) |
| I-12 | $(CH_3)_3C$ | -CH(OH)- | 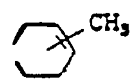 | | Öl |
| I-13 | $(CH_3)_3C$ | $-CH(OC_3H_7-n)-$ | 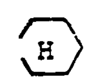 | | Öl |
| I-14 | $(CH_3)_3C$ | -CH(O-CO-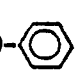)- | | 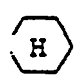 | Öl |
| I-15 | $(CH_3)_3C$ | $-CH(O-CO-CHCl_2)-$ | | 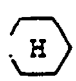 | Öl |
| I-16 | $(CH_3)_3C$ | $-CH(O-CO-N(CH_3)(SCCl_3))-$ | | 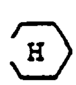 | Öl |
| I-17 | 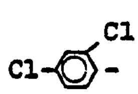 | -CH(OH)- | | 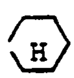 | Öl |

NDS = 1,5-Naphthalindisulfonsäure

Tabelle 1 (Fortsetzung)

| Bsp. Nr. | $R^1$ | $X^1$ | $R^2$ | $R^3$ | Schmelz-punkt (°C) |
|---|---|---|---|---|---|
| I-18 | 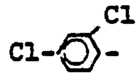 | -CH(OH)- | $C_2H_5$ | $C_2H_5$ | Öl |
| I-19 | 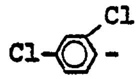 | -CH(OH)- | 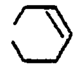 | | Öl |
| I-20 | 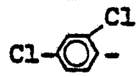 | $-CH(O-COCH_3)-$ | 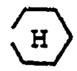 | | Öl |
| I-21 | $(CH_3)_3C$ | $-CCH_3(CH)-$ | 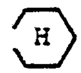 | | 101 |
| I-22 | $(CH_3)_3C$ | -CH(OH)- | 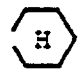 | | 154 (· HCl) (Z-Form) |
| I-23 | 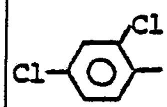 | -CH(OH)- | $C_3H_7$ | $CH_3$ | Öl |
| I-24 | $(CH_3)_3C$ | -CH(OH)- | 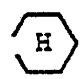 | | 110 ($\cdot CuCl_2$) (Z-Form) |
| I-25 | 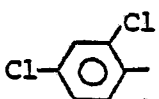 | $-CH(O-CO-NHCH_3)-$ | 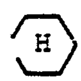 | | 62 |
| I-26 | 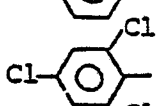 | -CH(OH)- | $C_2H_5$ | $CH_3$ | Öl |
| I-27 | 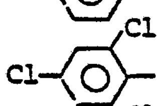 | 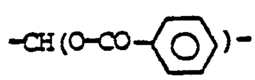 | $C_3H_7$ | $CH_3$ | Öl |
| I-28 | 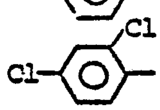 | $-CH(O-CO-CH_3)-$ | $C_3H_7$ | $CH_3$ | Öl |
| I-29 | $(CH_3)_3C$ | 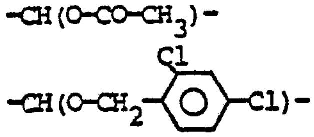 | 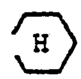 | | Öl (Z-Form) |
| I-30 | $ClCH_2-C(CH_3)_2-$ | -CO- | 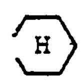 | | 51 |
| I-31 | $ClCH_2-C(CH_3)_2-$ | -CO- | 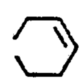 | | Öl |
| I-32 | $ClCH_2-C(CH_3)_2-$ | -CH(OH)- | 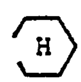 | | Öl |
| I-33 | 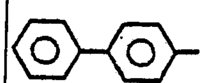 | -CH(OH)- | 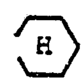 | | 156 |
| I-34 | $(CH_3)_3C$ | -CH(OH)- | 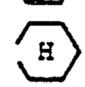 | | 153 ($\cdot HNO_3$) (Z-Form) |
| I-35 | 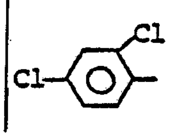 | -CH(OH)- | 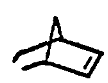 | | Öl |

Tabelle 1 (Forsetzung)

| Beispiel Nr. | $R^1$ | $X^1$ | $R^2$ | $R^3$ | Schmelzpunkt (°C) |
|---|---|---|---|---|---|
| I-36 | 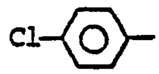 | $-CH(OH)-$ | 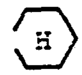 | | Öl |
| I-37 | 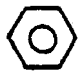 | $-CH(OH)-$ | 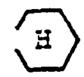 | | Öl |
| I-38 | 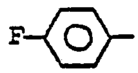 | $-CH(OH)-$ | $C_2H_5$ | $CH_3$ | Öl |
| I-39 | 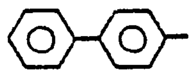 | $-CH(OH)-$ | $C_2H_5$ | $CH_3$ | Öl |
| I-40 | 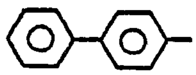 | $-CH(OH)-$ | $C_4H_9$ | $C_2H_5$ | Öl |
| I-41 | $ClCH_2-C(CH_3)_2-$ | $-CH(CH)-$ | 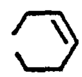 | | Öl |
| I-42 | $(CH_3)_3C$ | $-CH(OCH_3)-$ | 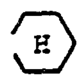 | | 63 (Z-Form) |
| I-43 | 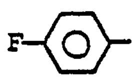 | $-CH(OH)-$ | $C_4H_9$ | $C_2H_5$ | Öl |
| I-44 | $FCH_2-C(CH_3)_2-$ | $-CO-$ | $C_4H_9$ | $C_2H_5$ | Öl |
| I-45 | $(CH_3)_3C$ | $-CH(OCH_3)-$ | 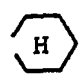 | | 104 (E-Form) |
| I-46 | $(CH_3)_3C$ | $-CH(OH)-$ | 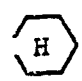 | | 137 (· $HNO_3$) (E-Form) |
| I-47 | 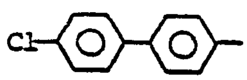 | $-CH(OH)-$ | $CH_3$ | $CH_3$ | 187 |
| I-48 | $ClCH_2-C(CH_3)_2-$ | $-CH(OH)-$ | $CH_3$ | $CH_3$ | Öl |
| I-49 | $(CH_3)_3C$ | $-CH(OH)-$ | 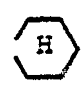 | | 242 (· $\frac{1}{2}$ NDS) (E-Form) |
| I-50 | $(CH_3)_3C$ | $-CH(CH)-$ | 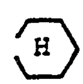 | | 168 (· $CuCl_2$) (E-Form) |
| I-51 | $(CH_3)_3C$ | $-CO-$ | 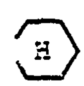 | | 137-140 (· $CuCl_2$) (E-Form) |

NDS = 1,5-Naphthalindisuldonsäure

Tabelle 1 (Fortsetzung)

| Beispiel Nr. | $R^1$ | $X^1$ | $R^2$ | $R^3$ | Schmelzpunkt (°C) |
|---|---|---|---|---|---|
| I-52 | Cl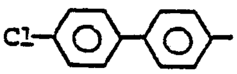 | $-CH(OH)-$ | 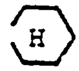 | | 157 |
| I-53 | Cl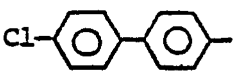 | $-CH(OH)-$ | $C_4H_9$ | $C_2H_5$ | 118 |
| I-54 | $FCH_2-C(CH_3)_2-$ | $-CO-$ | 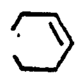 | | Öl |
| I-55 | $FCH_2-C(CH_3)_2-$ | $-CO-$ | 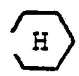 | | Öl |
| I-56 | $FCH_2-C(CH_3)_2-$ | $-CH(OH)-$ | 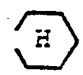 | | Öl |
| I-57 | $FCH_2-C(CH_3)_2-$ | $-CH(OH)-$ | 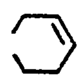 | | Öl |
| I-58 | $FCH_2-C(CH_3)_2-$ | $-CO-$ | 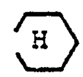 | | Öl (Z-Form) |
| I-59 | $FCH_2-C(CH_3)_2-$ | $-CO-$ | 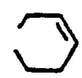 | | Öl (Z-Form) |
| I-60 | $ClCH_2-C(CH_3)_2-$ | $-CO-$ | 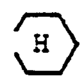 | | 103 (E-Form) |
| I-61 | Cl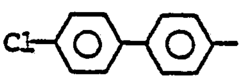 | $-CH(OH)-$ | | | 144 |
| I-62 | Cl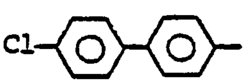 | $-CH(OH)-$ | $C_2H_5$ | $C_2H_5$ | 148 |
| I-63 | $FCH_2-C(CH_3)_2-$ | $-CH(OH)-$ | 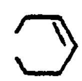 | | $n_D^{20}$: 1,5049 (Z-Form) |
| I-64 | $FCH_2-C(CH_3)_2-$ | $-CH(OH)-$ | 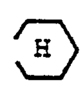 | | $n_D^{20}$: 1,4910 (Z-Form) |
| I-65 | $ClCH_2-C(CH_3)_2-$ | $-CH(OH)-$ | 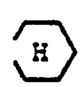 | | $n_D^{20}$: 1,5050 (E-Form) |

E- und Z-Form = Die beiden möglichen geometrischen isomeren Formen

Beispiel II-1

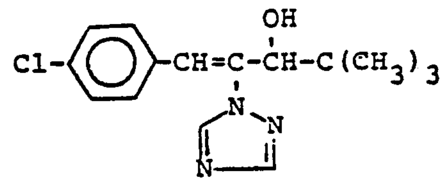

B-Form

Die Verbindung wird nach der in DE-A 2 920 437 angegebenen Methode hergestellt.

Beispiel II-2

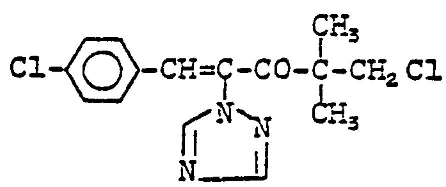

E/Z-Isomerengemisch

Beispiel II-3

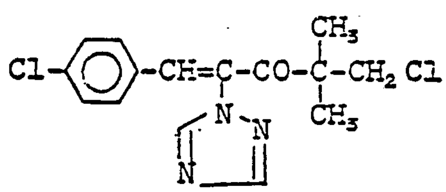

E-Isomeres

210,5 g (1 Mol) 1-Chlor-2,2-dimethyl-4-(1,2,4-triazol-1-yl)-butan-3-on, 140,5 g (1 Mol) 4-Chlorbenzylaldehyd sowie 9,9 ml Piperidin und 30 g Eisessig werden in 300 ml Toluol 8 Stunden unter Rückfluß erhitzt, wobei das entstehende Reaktionswasser azeotrop entfernt wird. Danach wird das Reaktionsgemisch mit Wasser und mit verdünnter Natriumhydrogencarbonatlösung gewaschen, über Natriumsulfat getrocknet und im Vakuum eingeengt. Man erhält 305,9 g (94,4 % der Theorie) rohes 1-Chlor-5-(4-chlorphenyl)-2,2-dimethyl-4-(1,2,4-triazol-1-yl)-4-penten-3-on vom Siedepunkt 165 °C/0,13 mbar als E,Z-Isomerengemisch. Durch Verrühren mit Isopropanol oder Ethanol läßt sich das E-Isomere in kristalliner Form isolieren. Es besitzt einen Schmelzpunkt von 112 °C.

Beispiel II-4

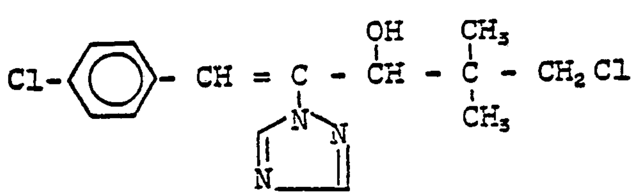

E/Z-Isomerengemisch

162 g (0,5 Mol) 1-Chlor-5-(4-chlorphenyl)-2,2-dimethyl-4-(1,2,4-triazol-1-yl)-4-penten-3-on (Beispiel III-2) werden in 500 ml Isopropanol gelöst und unter Rühren portionsweise mit 9,5 g (0,25 Mol) Natriumborhydrid versetzt. Man läßt das Reaktionsgemisch 10 Stunden bei Raumtemperatur rühren und engt anschließend im Vakuum ein. Der Rückstand wird in Toluol aufgenommen, mit verdünnter Essigsäure und anschließend mit Wasser gewaschen, über Natriumsulfat getrocknet und im Vakuum eingegeengt. Man erhält 156,6 g (96 % der Theorie) 1-Chlor-5-(4-chlorphenyl)-2,2-dimethyl 4-(1,2,4-triazol-1-yl)-4-penten-3-ol vom Brechungsindex $N_D^{20}$ = 1,557 9 als E/Z-Isomerengemisch.

Beispiel II-5

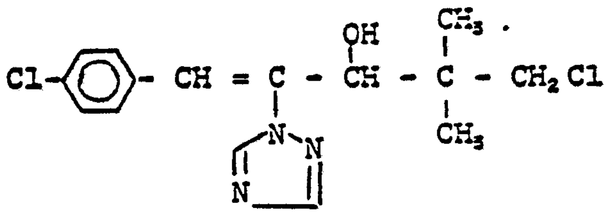

Z-Isomeres

48,6 g (0,15 Mol) 1-Chlor-5-(4-chlorphenyl)-2,2-dimethyl-4-(1,2,4-triazol-1-yl)-4-penten-3-on (Beispiel III-2) und 30,6 g (0,15 Mol) Aluminiumisopropylat werden in 200 ml Isopropanol 7 Stunden unter Rückfluß erhitzt, wobei über eine 30 cm Vigreux-Kolonne kontinuierlich Isopropanol und Aceton abdestilliert bis im Destillat kein Aceton mehr nachzuweisen ist. Danach wird das Reaktionsgemisch eingeengt und der Rückstand mit Eis/Salzsäure versetzt. Man extrahiert mit Methylenchlorid. Die vereinigten Methylenchloridextrakte werden über Natriumsulfat getrocknet und im Vakuum eingeengt. Der ölige Rückstand wird säulenchromatographisch (Kieselgel/Chloroform) getrennt. Man erhält das nicht reduzierte E-Isomere des 1-Chlor-5-(4-chlorphenyl)-2,2-dimethyl-4-(1,2,4-triazol-1-yl)-penten-3-ons (Beispiel 2) sowie reines 1-Chlor-5-(4-chlorphenyl)-2,2-dimethyl-4-(1,2,4-triazol-1-yl)-penten-3-ol vom Schmelzpunkt 120 °C als Z-Isomeres.

Beispiel II-6

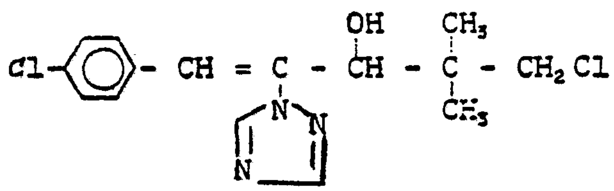

E-Isomeres

2,0 g (6,13 Mol) E-Isomeres des 1-Chlor-5-(4-chlorphenyl)-2,2-dimethyl-4-(1,2,4-triazol-1-yl)-4-penten-3-ons (Beispiel III-3) und 0,467 g (4,11 mMol) Calciumchlorid werden in 30 ml Isopropanol gelöst und bei – 5 °C tropfenweise mit einer Lösung von 0,167 g (4,3 mMol) Natriumborhydrid versetzt. Nach 6 Stunden wird das Reaktionsgemisch auf 25 °C erwärmt und im Vakuum eingeengt. Der Rückstand wird auf Wasser gegossen und mit Essigsäureethylester extrahiert. Die vereinigten Essigesterextrakte werden über Natriumsulfat getrocknet und im Vakuum eingeengt. Der ölige Pückstand kristallisiert beim Verrühren mit Diisopropylether. Man erhält 1,1 g (55 % der Theorie) 1-Chlor-5-(4-chlorphenyl)-2,2-dimethyl-4-(1,2,4-triazol-1-yl)-4-penten-3-ol vom Schmelzpunkt 170 °C als E-Isomeres.

In entsprechender Weise werden die folgenden Verbindungen der allgemeinen Formel (II) erhalten :

Tabelle 2

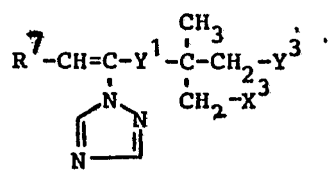

(IIb)

| Beispiel Nr. | $R^7$ | $Y^1$ | $X^3$ | $Y^3$ | Schmelzpunkt (°C) bzw. Brechungsindex ($n_D^{20}$) |
|---|---|---|---|---|---|
| II-7 | 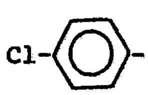 | CO | H | F | 1,5672 |
| II-8 | 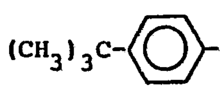 | CO | H | F | 1,5565 |
| II-9 | 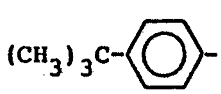 | CO | H | Cl | 1,5655 (Z-Isom.) |

Tabelle 2 (Fortsetzung)

| Beispiel Nr. | $R^7$ | $Y^1$ | $X^3$ | $Y^3$ | Schmelzpunkt (°C) bzw. Brechungsindex ($n_D^{20}$) |
|---|---|---|---|---|---|
| II-10 | 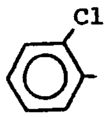 | CO | H | F | 1,5632 (E-Isom.) |
| II-11 | 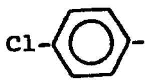 | CH(OH) | H | F | 230 (xNDS) |
| II-12 | 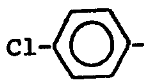 | CH(OH) | H | F | 110 (Z-Isom.) |
| II-13 | 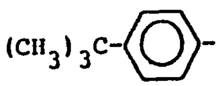 | CH(OH) | H | F | 1,5250 |
| II-14 | 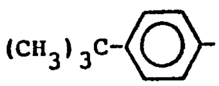 | CH(OH) | H | Cl | 1,5820 (Z-Isom.) |
| II-15 | 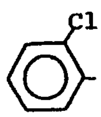 | CH(OH) | H | F | 116 (Z-Isom.) |
| II-16 | 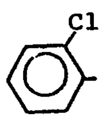 | CH(OH) | H | F | 140 (E-Isom.) |
| II-17 | 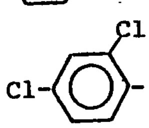 | CH(OH) | H | F | 150 (Z-Isom.) |
| II-18 | 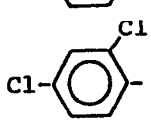 | CH(OH) | H | F | 158 (E-Isom.) |
| II-19 | 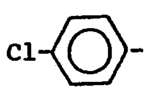 | CO | F | F | 1,5728 |
| II-20 | 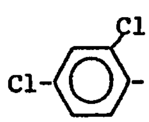 | CO | F | F | 1,5778 |
| II-21 | 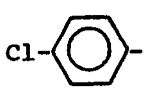 | CO | Cl | Cl | 1,5942 |
| II-22 | 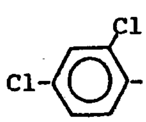 | CO | Cl | Cl | 1,5868 |
| II-23 | 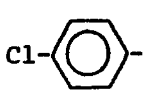 | CH(OH) | F | F | 74 (E-Isom.) |

NDS = 1,5-Naphthalindisulfonsäure

Tabelle 2 (Fortsetzung)

| Beispiel Nr. | $R^7$ | $Y^1$ | $X^3$ | $Y^3$ | Schmelzpunkt (°C) bzw. Brechungsindex ($n_D^{20}$) |
|---|---|---|---|---|---|
| II-24 | 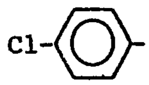 | CH(OH) | F | F | 110 (Z-Isom.) |
| II-25 | 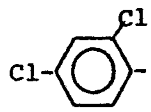 | CH(OH) | F | F | 157 (E-Isom.) |
| II-26 | 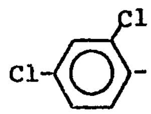 | CH(OH) | F | F | 154 (Z-Isom.) |
| II-27 | 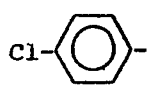 | CH(OH) | Cl | Cl | 150 (Z-Isom.) |
| II-28 | 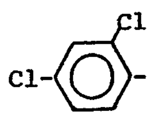 | CH(OH) | Cl | Cl | 148 (Z-Isom.) |

Beispiel (III-1)

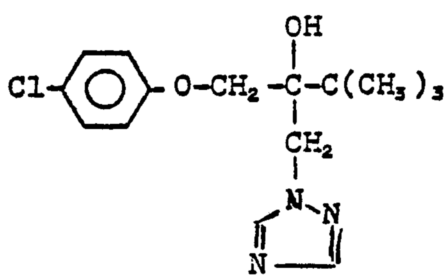

72,15 g (0,3 Mol) 2-(4-Chlorphenoxy-methyl)-2-tert.-butyl-oxiran und 24,15 g (0,33 Mol) 1,2,4-Triazol werden in 120 ml Ethanol 48 Stunden unter Rückfluß erhitzt. Anschließend wird eingeengt, der Rückstand in 200 ml Essigester aufgenommen und erhitzt. Danach wird im Eisbad abgekühlt, der Feststoff abgesaugt und mit Essigester nachgewaschen. Das Filtrat wird eingeengt, der Rückstand in Ether/Hexan gelöst und mit Chlorwasserstoff begast. Man saugt den Niederschlag ab, wäscht mit Ether nach und erhält durch Zugabe von Essigester/l n Natronlauge die freie Base. Man erhält 60,2 g (65 % der Theorie) 2-(4-Chlorphenoxy-methyl)-3,3-dimethyl-1-(1,2,4-triazol-1-yl)-butan-2-ol vom Schmelzpunkt 84-87 °C.

Herstellung des Ausgangsproduktes

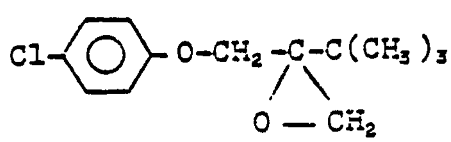

Eine Lösung von 189 ml (2,0 Mol) Dimethylsulfat in 1 200 ml absolutem Acetonitril wird bei Raumtemperatur mit einer Lösung von 162 ml (2,2 Mol) Dimethylsulfid in 400 ml absolutem Acetonitril versetzt. Man läßt die Reaktionsmischung über Nacht bei Raumtemperatur rühren. Danach werden 118,8 g (2,2 Mol) Natriummethylat zugegeben. Man läßt 30 Minuten rühren und versetzt dann innerhalb

von 30 Minuten tropfenweise mit einer Lösung von 272 g (1,2 Mol) 1-(4-Chlorphenoxy)-3,3-dimethyl-butan-2-on in 600 ml absolutem Acetonitril. Man läßt das Reaktionsgemisch über Nach nachrühren. Anschließend wird eingeengt, die organische Phase abgetrennt, zweimal mit Wasser und einmal mit gesättigter Natriumchloridlösung gewaschen, über Natriumsulfat getrocknet, eingeengt und der Rückstand im Vakuum destilliert. Man erhält 242,4 g (84 % der Theorie) 2-(4-Chlorphenoxy-methyl)-2-tert.-butyl-oxiran vom Siedepunkt 115-22 °C/0,004 mbar und vom Schmelzpunkt 50-52 °C.

Beispiel (III-2)

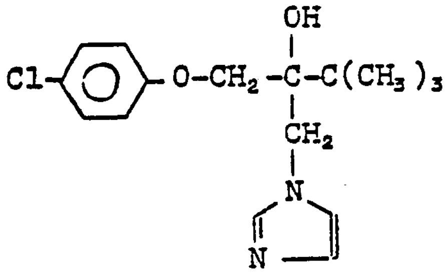

2,71 g (0,117 8 Mol) Natrium in 250 ml absolutem Ethanol werden mit 8,02 g (0,117 8 Mol) Imidazol versetzt. Innerhalb von 30 Minuten läßt man bei Raumtemperatur eine Lösung von 14,17 g (0,058 9 Mol) 2-(4-Chlorphenoxymethyl)-2-tert-butyl-oxiran in 100 ml Ethanol zutropfen. Danach wird das Reaktionsgemisch 8 Stunden unter Rückfluß erhitzt, eingeengt und der Rückstand in Ether aufgenommen. Man extrahiert dreimal mit 1 n Salzsäure, neutralisiert die vereinigten Salzsäurephasen mit Natriumhydrogencarbonat und extrahiert anschließend mit Essigester. Nach dem Einengen und Umkristallisieren aus Cyclohexan erhält man 11,6 g (64 % der Theorie) 2-(4-Chlorphenoxy-methyl)-3,3-dimethyl-1-(imidazol-1-yl)-butan-2-ol vom Schmelzpunkt 154-55 °C.

Beispiel (III-3)

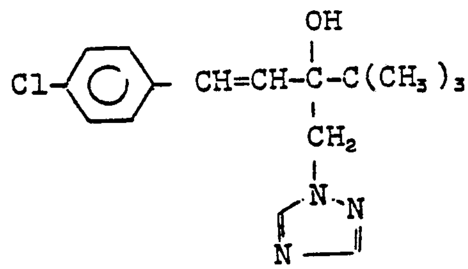

Eine Lösung von 17,75 g (0,075 Mol) 2-(4-Chlorphenyl-ethenyl)-2-tert-butyl-oxiran und 6,9 g (0,1 Mol) 1,2,4-Triazol in 30 ml Ethanol wird 20 Stunden bei 150 °C im Bombenrohr erhitzt. Danach wird das Reaktionsgemisch eingeengt und der kristalline Rückstand mit Ether verrührt. Der Feststoff wird anschließend abgesaugt und aus Acetonitril umkristallisiert. Man erhält 17,7 g (77 % der Theorie) 1-(4-Chlorphenyl)-4,4-dimethyl-3-(1,2,4-triazol-1-yl-methyl)-1-penten-3-ol vom Schmelzpunkt 139-41 °C.

Beispiel (III-4)

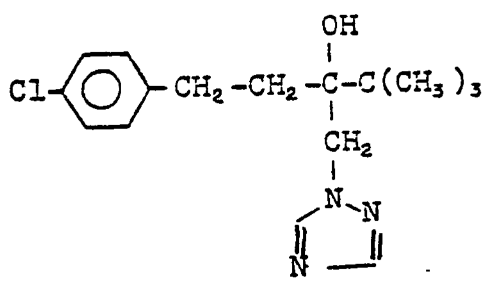

Eine Lösung von 17,9 g (0,075 Mol) 2-(4-Chlorphenyl-ethyl)-2-tert.-butyl-oxiran und 6,9 g (0,1 Mol) 1,2,4-Triazol in 30 ml Ethanol wird 20 Stunden bei 150 °C im Bombenrohr erhitzt. Man läßt abkühlen und engt die Reaktionslösung ein. Der Rückstand wird in Ether gelöst, dreimal mit Wasser und einmal mit Natriumchloridlösung gewaschen, über Natriumsulfat getrocknet und eingeengt. Der Rückstand wird

über eine Kieselgelsäule chromatographiert (Laufmittel: Dichlormethan/Essigester 1 : 1. Man erhält 12,3 g (53,2 % der Theorie) 1-(4-Chlorphenyl)-4,4-dimethyl-3-(1,2,4-triazol-1-yl-methyl)-pentan-3-ol als zähflüssiges Oel.

In analoger Weise wurden die nachfolgenden Verbindungen der allgemeinen Formel (III) erhalten :

Tabelle 3

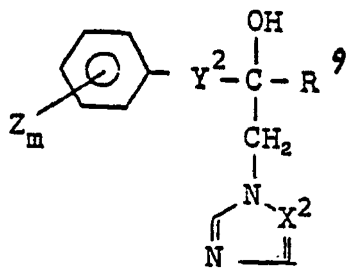

| Bsp. Nr. | $Z_m$ | $Y^2$ | $R^9$ | $X^2$ | Schmelzpunkt (°C) |
|---|---|---|---|---|---|
| III-5 | 4-Cl, 2-$CH_3$ | $-O-CH_2-$ | $-C(CH_3)_3$ | N | 125,5-29 |
| III-6 | 2,4-$Cl_2$ | $-O-CH_2-$ | $-C(CH_3)_3$ | N | 120,5-23,5 |
| III-7 | 4-$CH_3$ | $-O-CH_2-$ | $-C(CH_3)_3$ | N | 98-101,5 |
| III-8 | 2-$CH_3$ | $-O-CH_2-$ | $-C(CH_3)_3$ | N | 89-101 |
| III-9 | 4-F | $-CH_2-CH_2-$ | $-C(CH_3)_3$ | N | 91-95,5 |
| III-10 | 2-$CH_3$ | $-CH{=}CH-$ | $-C(CH_3)_3$ | N | Oel |
| III-11 | 4-Cl | $-CH_2-CH_2-$ | $-C(CH_3)_3$ | N | 212(Zers.) (xHCl) |
| III-12 | 2,4-$Cl_2$ | $-O-CH_2-$ | $-C(CH_3)_3$ | CH | 152-54 |
| III-13 | 4-$CH_3$ | $-O-CH_2-$ | $-C(CH_3)_3$ | CH | 129-31 |
| III-14 | 2-$CH_3$ | $-O-CH_2-$ | $-C(CH_3)_3$ | CH | 123-24 |
| III-15 | 4-Cl, 2-$CH_3$ | $-O-CH_2-$ | $-C(CH_3)_3$ | CH | 157-59 |
| III-16 | 4-Cl | $-CH_2-CH_2-$ | $-C(CH_3)_3$ | CH | 157,5-59,5 |
| III-17 | 4-F | $-CH_2-CH_2-$ | $-C(CH_3)_3$ | CH | 124-25 |
| III-18 | 2-$CH_3$ | $-CH_2-CH_2-$ | $-C(CH_3)_3$ | CH | 94-99 |
| III-19 | 4-Cl | $-CH{=}CH-$ | $-C(CH_3)_3$ | CH | 158,5-62 |
| III-20 | 4-F | $-CH{=}CH-$ | $-C(CH_3)_3$ | CH | 144-46 |
| III-21 | 2-$CH_3$ | $-CH{=}CH-$ | $-C(CH_3)_3$ | CH | 127-32 |
| III-22 | 4-Cl | $-O-CH_2-$ | 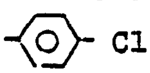 | CH | 216-17 (x 1/2 NDS)* |
| III-23 | 4-$CH_3$ | $-CH{=}CH-$ | $-C(CH_3)_3$ | N | 117-19 |
| III-24 | 4-$CH_3$ | $-CH{=}CH-$ | $-C(CH_3)_3$ | CH | 144-46 |
| III-25 | 2,6-$Cl_2$ | $-CH{=}CH-$ | $-C(CH_3)_3$ | CH | 110-16 |
| III-26 | 4-$CH_3$ | $-CH_2-CH_2-$ | $-C(CH_3)_3$ | N | Öl |

NDS = 1,5-Naphthalindisulfonsäure

Beispiel (IV-1)

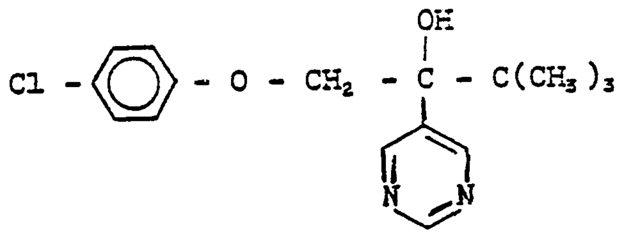

Eine Lösung von 22,65 g (0,1 Mol) 1-(4-Chlorphenoxy)-3,3-dimethyl-butan-2-on in 110 ml absolutem Tetrahydrofuran und 70 ml absolutem Ether wird unter trockener Stickstoffatmosphäre auf – 120 °C abgekühlt. Dazu tropft man eine Lösung von 15,9 g (0,1 Mol) 5-brompyrimidin in 50 ml absolutem Tetrahydrofuran. Anschließend werden bei – 120 °C 50 ml einer 15 %-igen Lösung von n-Butyllithium in n-Hexan langsam zugetropft. Man läßt zunächst 2 Stunden bei einer Temperatur von ca. – 110 °C, danach über Nacht bei – 78 °C nachrühren. Das Reaktionsgemisch wird auf Raumtemperatur erwärmt, mit 100 ml 10 %iger Ammoniumchlorid-Lösung und 200 ml Essigester versetzt und die wässrige Phase abgetrennt. Die organische Phase wird nacheinander einmal mit 1 n Salzsäure und zweimal gesättigter Natriumchloridlösung gewaschen, über Natriumsulfat getrocknet und eingeengt. Der Rückstand wird mit Ether aufgeschlämmt, der Feststoff agbesaugt und aus Acetonitril umkristallisiert. Man erhält 12,3 g (50 % der Theorie bezogen auf n-Butyllithium) 1-(4-Chlorphenoxy)-3,3-dimethyl-2-(pyrimidin-5-yl)-butan-2-ol vom Schmelzpunkt 172-174 °C.

Analog werden die in der folgenden Tabelle 4 aufgeführten Verbindungen der Formel

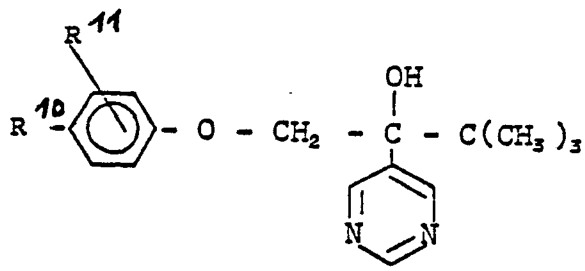

(IV)

erhalten :

Tabelle 4

| Bsp.Nr. | $R^{10}$ | $R^{11}$ | Schmelzpunkt(°C) |
|---|---|---|---|
| IV-2 | H | H | 127-29 |
| IV-3 | $CH_3O-$ | H | 136-37 |
| IV-4 | F | H | 163,5-64,5 |
| IV-5 | Cl | 2-Cl | 96-99 |
| IV-6 | Cl | 3-Cl | 155-57 |
| IV-7 | $CH_3$ | H | 152-53,5 |
| IV-8 | 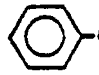 $-CH_2-O-$ | H | 122-24 |

Beispiel (VI-1)

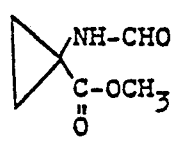

Zu 40 ml Wasser werden bei 20 °C nacheinander 0,3 ml konzentrierte Salzsäure und eine Lösung von 5 g (0,04 Mol) α-Isocyano-cyclopropancarbonsäure-methyl-ester in 10 ml Methanol gegeben. Die Reaktionsmischung wird 6 Stunden gerührt. Dann extrahiert man zweimal mit je 50 ml Methylenchlorid, trocknet die organische Phase über Magnesiumsulfat, filtriert und destilliert das Lösungsmittel im Vakuum ab. Als Rückstand verbleiben in Form einer farblosen Flüssigkeit 4 g (70 % der Theorie) α-Formylamino-cyclopropancarbonsäure-methyl-ester. Brechungsindex : $n_D^{20}$ = 1,473 0.

Beispiel (VI-2)

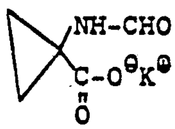

Eine Lösung von 7 g (0,05 Mol) α-Isocyano-cyclopropancarbonsäure-äthylester in 100 ml Äther wird bei 5 °C tropfenweise mit einer Lösung von 3,1 g (0,55 Mol) Kaliumhydroxid in 50 ml Äthanol versetzt. Die Mischung wird 12 Stunden bei 20 °C gerührt. Nach Absaugen und Waschen mit Äther erhält man als weißes Pulver 6,4 g (86 % der Theorie) α-Isocyano-cyclopropancarbonsäure-Kaliumsalz. Schmelzpunkt : 225 °C.

Eine Suspension von 9 g (0,06 Mol) α-Isocyano-cyclopropan-carbonsäure-Kaliumsalz in 50 ml Äthanol wird mit 1,18 g (0,066 Mol) Wasser versetzt. Die Mischung wird 12 Stunden unter Rückfluß zum Sieden erhitzt und nach dem Abkühlen bei 20 °C mit 50 ml Äther versetzt. Nach Absaugen erhält man in Form weißer Kristalle 7 g (70 % der Theorie) α-Formylaminocyclopropancarbonsäure-Kaliumsalz. Schmelzpunkt : 186 °C

Beispiel (VI-3)

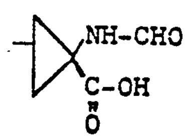

8,36 g (0,05 Mol) α-Formylamino-cyclopropancarbonsäure-Kaliumsalz werden in 20 ml Wasser gelöst und bei 0 °C mit 5 g (0,05 Mol) konzentrierter Salzsäure versetzt. Die Mischung wird über Nacht bei 5 °C stehen gelassen. Nach Absaugen und Trocknen erhält man in Form farbloser Kristalle 5,2 g (80 % der Theorie) α-Formyl-amino-cyclopropancarbonsäure. Schmelzpunkt : 189 °C.

Beispiel (VI-4)

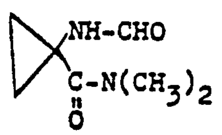

Zu einer Lösung von 6,7 g (0,15 Mol) Dimethylamin in 50 ml Wasser gibt man bei 20 °C unter Rühren 7,46 g (0,05 Mol) α-Isocyano-cyclopropancarbonsäure-Kaliumsalz. Nach Abkühlen auf 5 °C wird die Reaktionsmischung 5 g (0,05 Mol) konzentrierter Salzsäure versetzt und im verschlossenen Reaktionsgefäß 12 Stunden bei 20 °C stehen gelassen. Bei einer Badtemperatur von 60 °C werden die flüchtigen Komponenten im Wasserstrahlvakuum abgezogen. Aus dem Rückstand wird das Produkt mit Methylenchlorid extrahiert ; die Lösung wird mit Magensiumsulfat getrocknet und nach Filtration wird das Lösungsmittel im Vakuum abdestilliert. Es verbleiben 5,5 g (70 % der Theorie) α-Formylamino-cyclopropancarbonsäure-N,N-dimethylamid in Form einer hellgeben Flüssigkeit. Brechungsindex : $n_D^{20}$ = 1,435 0.

Beispiel (VI-5)

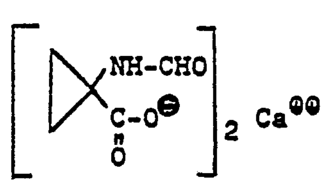

Eine Mischung aus 2,5 g (0,02 Mol) α-Formylamino-cyclopropancarbonsäure und 40 ml Äthanol wird unter Rühren bei 25 °C mit 0,74 g (0,01 Mol) Calciumhydroxid versetzt und man läßt anschließend 12 Stunden bei Raumtemperatur nachrühren. Die Lösung wird anschließend im Vakuum eingedampft und der Rückstand mit Äther verrieben. Nach Absaugen und Trocknen erhält man in Form eines weißen Pulvers 2,6 g (97 % der Theorie) α-Formylamino-cyclopropancarbonsäure-Calciumsalz. Schmelzpunkt : 290 °C.

Beispiel (VI-6)

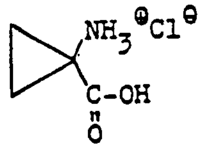

Eine Mischung aus 19,4 g (0,15 Mol) α-Formylaminocyclopropan-carbonsäure und 200 ml 18 %iger Salzsäure wird 3 Stunden unter Rückfluß zum Sieden erhitzt. Anschließend wird im Vakuum zur Trockne eingedampft und der zurückbleibende Feststoff über Phosphorpentoxid im Vakuumexsikkator getrocknet.

Ausbeute : 18 g (92 % der Theorie) α-Amino-cyclopropancarbonsäure-hydrochlorid. Schmelzpunkt : 232 °C.

Beispiel (VI-7)

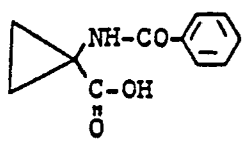

Eine Mischung aus 2 g (0,02 Mol) α-Amino-cyclopropancarbonsäure, 25 ml Wasser und 2,55 g (0,044 Mol) Kaliumhydroxid wird unter Rühren bei 20 °C mit 3,1 g (0,022 Mol) Benzoylchlorid versetzt. Nach 30-minütigem Rühren wird mit konzentrierter Salzsäure auf pH 1 angesäuert und abgesaugt. Zur Reinigung wird das Produkt mit 30 ml Wasser ausgekocht.

Ausbeute : 2,1 g (51 % der Theorie) α-Benzylaminocyclopropancarbonsäure. Schmelzpunkt 209 °C.

In analoger Weise werden die in der nachstehenden Tabelle 5 aufgeführten Verbindungen der Formel

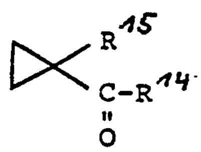

hergestellt :

Tabelle 5

| Beispiel Nr. | $R^{14}$ | $R^{15}$ | Ausbeute (% der Theorie) | Brechungsindex $n_D^{20}$; Schmelzpunkt (°C); bzw. Siedepunkt (°C/mbar) |
|---|---|---|---|---|
| VI—8 | 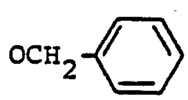 | NHCHO | 74 | 1,5079 |
| VI—9 | $O^{\ominus}HN(C_2H_5)_3^{\oplus}$ | NHCHO | 85 | 1,4461 |

Tabelle 5 (Fortsetzung)

| Beispiel Nr. | $R^{14}$ | $R^{15}$ | Ausbeute (% der Theorie) | Brechungsindex $n_D^{20}$; Schmelzpunkt (°C); bzw. Siedepunkt (°C/mbar) |
|---|---|---|---|---|
| VI-10 | $OC_2H_5$ | NHCHO | 71 | 110/0,1 |
| VI-11 | $NH_2$ | NHCHO | 70 | 145 |
| VI-12 | $OCH_3$ | $NH_3^{\oplus}Cl^{\ominus}$ | 81 | 180 |
| VI-13 | $OCH_3$ | $NH_2$ | 77 | 1,4491 |
| VI-14 | $OCH_2$-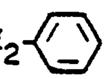 | $NH_3^{\oplus}Cl^{\ominus}$ | 51 | 92 |
| VI-15 | $OCH_2$-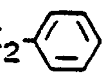 | $NH_2$ | 86 | 1,4849 |
| VI-16 | $O^{\ominus}Na^{\oplus}$ | $NH_2$ | 97 | 216 |
| VI-17 | OH | $NH_2$ | 75 | 220 |
| VI-18 | $OC_2H_5$ | $NH_3^{\oplus}Cl^{\ominus}$ | 83 | 108 |
| VI-19 | $OC_2H_5$ | $NH_2$ | 75 | 1,4440 |
| VI-20 | $O^{\ominus}K^{\oplus}$ | $NHCOCH_3$ | 82 | 246 |
| VI-21 | $OC_2H_5$ | $NHCOCH_3$ | 90 | 76 |
| VI-22 | $O(CH_2)_7CH_3$ | NHCHO | 95 | 1,4321 |
| VI-23 | $O(CH_2)_7CH_3$ | $NH_3^{\oplus}Cl^{\ominus}$ | 91 | 1,4429 |
| VI-24 | $O^{\ominus}Na^{\oplus}$ | NHCHO | 90 | 221 |

**Ansprüche**

1. Mittel zur Hemmung des Pflanzenwachstums, gekennzeichnet durch einen Gehalt an einer Wirkstoffkombination bestehend aus

A) einem Azol der Formel

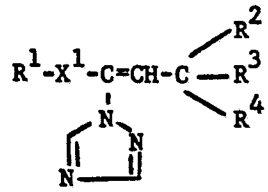

(I)

in welcher

$R^1$ für gegebenenfalls substituiertes Alkyl, Cycloalkyl oder gegebenenfalls substituiertes Aryl steht,

$R^2$ für Wasserstoff oder Alkyl steht,

$R^3$ für Wasserstoff, Alkyl, Cycloalkyl, gegebenenfalls substituiertes Cycloalkenyl, Alkenyl oder gegebenenfalls substituiertes Aryl steht,

$R^2$ und $R^3$ gemeinsam mit dem Kohlenstoffatom, an das sie gebunden sind, für gegebenenfalls substituiertes Cycloalkenyl oder Cycloalkyl stehen,

$R^4$ für Wasserstoff oder Alkyl steht,

$X^1$ für die Gruppe

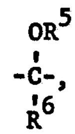

steht, sowie zusätzlich für die Ketogruppe steht, wenn $R^1$ für gegebenenfalls substituiertes Alkyl oder Cycloalkyl steht,

$R^5$ für Wasserstoff, Alkyl, gegebenenfalls substituiertes Aralkyl, Acyl oder gegebenenfalls substituiertes Carbamoyl steht und

$R^6$ für Wasserstoff, Alkyl oder gegebenenfalls substituiertes Aralkyl steht,

oder einem Azol der Formel

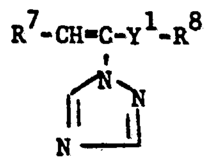

(II)

in welcher

$R^7$ für gegebenenfalls substituiertes Aryl steht,

$R^8$ für Alkyl, Halogenalkyl oder gegebenenfalls substituiertes Aryl steht und

$Y^1$ für Carbonyl oder für die Gruppe —CH(OH)— steht,

oder einem Azol-Derivat der Formel

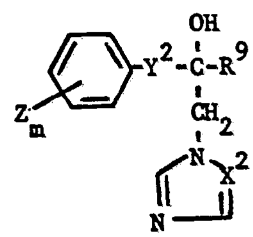

(III)

in welcher

$R^9$ für Alkyl, gegebenenfalls substituiertes Cycloalkyl oder gegebenenfalls substituiertes Phenyl steht,

$X^2$ für ein Stickstoffatom oder die CH-Gruppe steht,

$Y^2$ für die Gruppierungen $—OCH_2—$, $—CH_2CH_2—$ oder —CH=CH— steht, wobei im Falle der $—OCH_2$-Gruppe das Sauerstoffatom mit dem Phenylrest verbunden ist, für Halogen, Alkyl, Cycloalkyl, Alkoxy, Alkylthio, Halogenalkyl, Halogenalkoxy, Halogenalkylthio, gegebenenfalls substituiertes Phenyl, gegebenenfalls substituiertes Phenoxy, gegebenenfalls substituiertes Phenylalkyl oder gegebenenfalls substituiertes Phenylalkoxy steht und

m für die Zahlen 0, 1, 2 oder 3 steht,

oder einem Säureadditions-Salz oder Metallsalz-Komplex von Azolen der Formeln (I), (II) oder (III),

oder einem Pyrimidin-butanol-Derivat der Formel

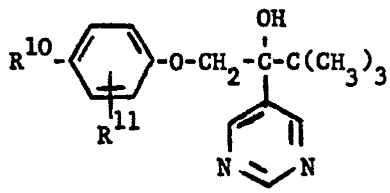

(IV)

in welcher

$R^{10}$ für Wasserstoff, Halogen, Alkyl, Alkoxy oder gegebenenfalls durch Halogen substituiertes Benzyloxy steht und

$R^{11}$ für Wasserstoff oder Halogen steht,

und

B) einem Phosphonsäure-Derivat der Formel

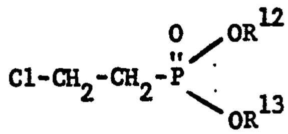

(V)

in welcher

$R^{12}$ und $R^{13}$ unabhängig voneinander für Wasserstoff oder ein Alkalimetall-Kation stehen,

oder einem 1-Amino-cyclopropan-1-carbonsäure-Derivat der Formel

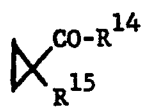

(VI)

in welcher

$R^{14}$ für Hydroxy, Alkoxy, Aralkoxy, Amino, Alkylamino, Dialkylamino oder für den Rest

$$O^{\ominus}M^{\oplus}$$

steht, wobei $M^{\oplus}$ für ein Alkali- oder Erdalkalimetallionenäquivalent oder für ein Ammonium-, Alkylammonium-, Dialkylammonium-, Trialkylammonium- oder Tetraalkylammoniumion steht, und

$R^{15}$ für Amino oder für den Rest —NH—CO—R steht, worin

R für Wasserstoff, Alkyl oder Aryl steht

und

$R^{15}$ ferner für den Rest $—N^{\oplus}H_3A^{\ominus}$ steht, wobei $A^{\ominus}$ für Chlorid, Bromid oder Jodid steht.

2. Mittel gemäß Anspruch 1, dadurch gekennzeichnet, daß in den Wirkstoffkombinationen das Gewischtsverhältnis von einem unter (A) aufgeführten Wirkstoff zu einem unter (B) aufgeführten Wirkstoff zwischen 1 : 0,05 und 1 : 5, vorzugsweise zwischen 1 : 0,1 und 1 : 4 liegt.

3. Verfahren zur Hemmung des Pflanzenwachstums, fadurch gekennzeichnet, daß man Wirkstoffkombinationen gemäß Anspruch 1 oder 2 auf die Pflanzen und/oder deren Lebensraum einwirken läßt.

4. Verwendung von Wirkstoffkombinationen gemäß Anspruch 1 oder 2 zur Hemmung des Pflanzenwachstums.

5. Verfahren zur Herstellung von Mitteln zur Hemmung des Pflanzenwachstums, dadurch gekennzeichnet, daß man Wirkstoffkombinationen gemäß Anspruch 1 oder 2 mit Streckmitteln und/oder oberflächenaktiven Mitteln vermischt.

**Claims**

1. Agent for inhibiting plant growth, characterised by a content of an active compound combination consisting of

A) an azole of the formula

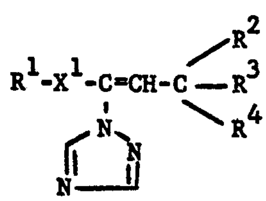

(I)

in which
$R^1$ represents optionally substituted alkyl, cycloalkyl or optionally substituted aryl,
$R^2$ represents hydrogen or alkyl,
$R^3$ represents hydrogen, alkyl, cycloalkyl, optionally substituted cycloalkenyl, alkenyl or optionally substituted aryl,
$R^2$ and $R^3$ together with the carbon atom to which they are bonded represent optionally substituted cycloalkenyl or cycloalkyl,
$R^4$ represents hydrogen of alkyl,
$X^1$ represents the group

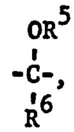

and additionally represents the keto group if $R^1$ represents optionally substituted alkyl or cycloalkyl,
$R^5$ represents hydrogen, alkyl, optionally substituted aralkyl, acyl or optionally substituted carbamoyl and
$R^6$ represents hydrogen, alkyl or optionally substituted aralkyl,
or an azole of the formula

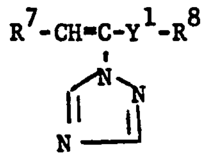

(II)

in which
$R^7$ represents optionally substituted aryl,
$R^8$ represents alkyl, halogenoalkyl or optionally substituted aryl and
$Y^1$ represents carbonyl or the group —CH(OH)—,
or an azole derivative of the formula

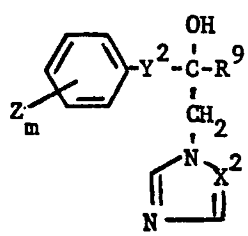

(III)

in which
$R^9$ represents alkyl, optionally substituted cycloalkyl or optionally substituted phenyl,
$X^2$ represents a nitrogen atom or the CH group,
$Y^2$ represents the groupings $—OCH_2—$, $—CH_2CH_2—$ or —CH=CH—, the oxygen atom being linked to the phenyl radical in the case of the $—OCH_2$-group,
Z represents halogen, alkyl, cycloalkyl, alkoxy, alkylthio, halogenoalkyl, halogenoalkoxy, halogenoalkylthio, optionally substituted phenyl, optionally substituted phenoxy, optionally substituted phenylalkyl or optionally substituted ohenylalkoxy and
m represents the numbers 0, 1, 2 or 3,
or an acid addition salt or metal salt complex of azoles of the formulae (I), (II) or (III),
or a pyrimidine butanol derivative of the formula

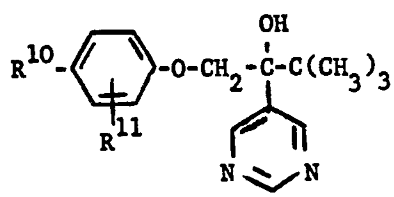

(IV)

in which
$R^{10}$ represents hydrogen, halogen, alkyl, alkoxy or benzyloxy which is optionally substituted by

halogen and

$R^{11}$ represents hydrogen or halogen, and

B) a phosphonic acid derivative of the formula

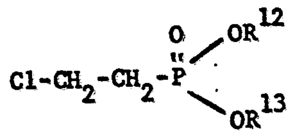

(V)

in which

$R^{12}$ and $R^{13}$ independently of one another represent hydrogen or an alkali metal cation, or a 1-amino-cyclopropane-1-carboxylic acid derivative of the formula

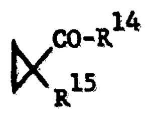

(VI)

in which

$R^{14}$ represents hydroxyl, alkoxy, aralkoxy, amino, alkylamino, dialkylamino or the radical

$$O^{\ominus}M^{\oplus}$$

wherein $M^{\oplus}$ represents an alkali metal ion equivalent or alkaline earth metal ion equivalent or an ammonium, alkylammonium, dialkylammonium, trialkylammonium or tetraalkylammonium ion, and

$R^{15}$ represents amino or the radical —NH—CO—R, wherein

R represents hydrogen, alkyl or aryl and

$R^{15}$ further represents the radical $—N^{\oplus}H_3A^{\ominus}$, wherein

$A^{\ominus}$ represents chloride, bromide or iodide.

2. Agent according to Claim 1, characterised in that the ratio by weight of an active compound listed under (A) to an active compound listed unter (B) is between 1: 0.05 and 1: 5, preferably between 1: 0.1 and 1: 4, in the active compound combinations.

3. Process for inhibiting plant growth, characterised in that active compound combinations according to Claim 1 or 2 are allowed to act on the plants and/or their habitat.

4. Use of active compound combinations according to Claim 1 or 2, for inhibiting plant growth.

5. Process for the preparation of agents for inhibiting plant growth, characterised in that active compound combinations according to Claim 1 or 2 are mixed with extenders and/or surface-active agents.

**Revendications**

1. Produit pour inhiber la croissance des plantes, caractérisé en ce qu'il contient une combinaison de substances actives consistant en

A) un azole de formule

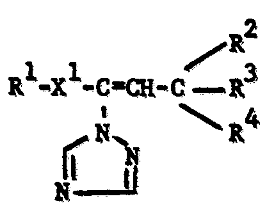

(I)

dans laquelle

$R^1$ représente un groupe alkyle éventuellement substitué, cycloalkyle ou aryle éventuellement substitué,

$R^2$ représente l'hydrogène ou un groupe alkyle,

$R^3$ représente l'hydrogène, un groupe alkyle, cycloalkyle, cycloalcényle éventuellement substitué, alcényle ou aryle éventuellement substitué,

$R^2$ et $R^3$, pris avec l'atome de carbone auquel ils sont reliés, représentent un groupe cycloalcényle éventuellement substitué, ou cycloalkyle,

$R^4$ représente l'hydrogène ou un groupe alkyle,

$X^1$ représente le groupe

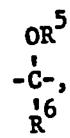

ainsi que, en outre, le groupe cétonique, lorsque $R^1$ représente un groupe alkyle éventuellement substitué ou cycloalkyle,

$R^5$ représente un atome d'hydrogène, un groupe alkyle, aralkyle éventuellement substitué, acyle ou carbamoyle éventuellement substitué, et

$R^6$ représente l'hydrogène, un groupe alkyle ou aralkyle éventuellement substitué,

ou un azole de formule

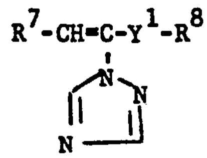

(II)

dans laquelle

$R^7$ représente un groupe aryle éventuellement substitué,

$R^8$ représente un groupe alkyle, halogénoalkyle ou aryle éventuellement substitué, et

$Y^1$ représente un groupe carbonyle ou le groupe —CH(OH)— ou un dérivé d'azole de formule

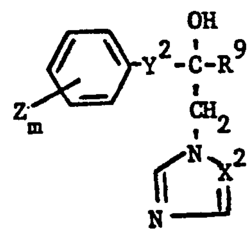

(III)

dans laquelle

$R^9$ représente un groupe alkyle, cycloalkyle éventuellement substitué ou phényle éventuellement substitué,

$X^2$ représente un atome d'azote ou le groupe CH,

$Y^2$ représente les groupements $—OCH_2—$, $—CH_2CH_2—$ ou —CH=CH—, et, dans le cas du groupe $—OCH_2—$, l'atome d'oxygène est relié au radical phényle,

Z représente un atome d'halogène, un groupe alkyle, cycloalkyle, alcoxy, alkylthio, halogénoalkyle, halogénoalcoxy, halogénoalkylthio, phényle éventuellement substitué, phénoxy éventuellement substitué, phénylalkyle éventuellement substitué ou phénylalcoxy éventuellement substitué, et

m représente les nombres 0, 1, 2 ou 3,

ou un sel d'addition d'acide ou un complexe de sel de métal d'azoles de formule (I), (II) ou (III), ou un dérivé de pyrimidine-butanol de formule :

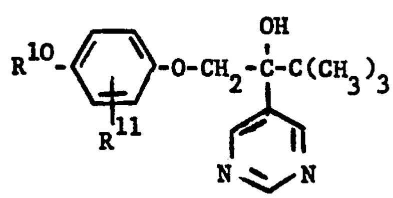

(IV)

dans laquelle

$R^{10}$ représente un atome d'hydrogène ou d'halogène, un groupe alkyle, alcoxy ou benzyloxy éventuellement substitué par de l'halogène, et

$R^{11}$ représente un atome d'hydrogène ou d'halogène, et

B) un dérivé d'acide phosphonique de formule

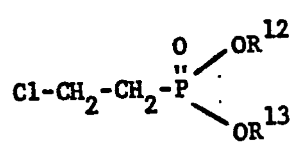

(V)

dans laquelle

$R^{12}$ et $R^{13}$ représentent, indépendamment l'un de l'autre, un atome d'hydrogène ou un cation de métal alcalin,

ou un dérivé d'acide 1-amino-cyclopropane-1-carboxylique de formule :

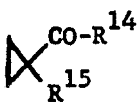

(VI)

dans laquelle

$R^{14}$ représente un groupe hydroxyle, alcoxy, aralcoxy, amino, alkylamino, dialkylamino ou le radical

$O^{\ominus}M^{\oplus}$

où $M^{\oplus}$ représente un équivalent d'un ion de métal alcalin ou alcalino-terreux ou un ion ammonium, alkylammonium, dialkylammonium, trialkylammonium ou tétraalkylammonium, et

$R^{15}$ représente un groupe amino ou le radical —NH—CO—R, où

R représente un atome d'hydrogène, un groupe alkyle ou aryle, et

$R^{15}$ représente en outre le radical $—N^{\oplus}H_3A^{\ominus}$, où $A^{\ominus}$ représente un anion chlorure, bromure ou iodure.

2. Produit selon la revendication 1, caractérisé en ce que, dans la combinaison de substances actives, le rapport pondéral entre une substance active indiquée en (A) et une substance active indiquée en (B) se situe entre 1 : 0,05 et 1 : 5, avantageusement entre 1 : 0,1 et 1 : 4.

3. Procédé pour inhiber la croissance de plantes, caractérisé en ce qu'on fait agir sur les plantes et/ou sur leur espace vital ou biotope des combinaisons de substances actives selon la revendication 1 ou 2.

4. Utilisation de combinaisons de substances actives selon la revendication 1 ou 2 pour inhiber la croissance de plantes.

5. Procédé de préparation de produits pour inhiber la croissance de plantes, caractérisé en ce que l'on mélange des combinaisons de substances actives selon la revendication 1 ou 2 avec des agents d'allongement et/ou des agents tensio-actifs.